# EUROPEAN PATENT SPECIFICATION

(11) **EP 2 771 024 B1**
(45) Date of publication and mention of the grant of the patent: **28.11.2018**
(21) Application number: 12775720.1
(22) Date of filing: 26.10.2012
(51) Int. Cl.: A61K 38/26, A61K 38/28, A61K 31/155, A61P 3/10, A61P 3/04

(54) **TREATMENT PROTOCOL OF DIABETES TYPE 2**
BEHANDLUNGSPROTOKOLL FÜR DIABETES TYP 2
PROTOCOLE DE TRAITEMENT DU DIABÈTE DE TYPE 2

(30) Priority: 28.10.2011 EP 11187169
(43) Date of publication of application: 03.09.2014
(73) Proprietor: Sanofi-Aventis Deutschland GmbH, 65929 Frankfurt am Main (DE)
(72) Inventor: SILVESTRE, Louise, F-75008 Paris (FR); SOUHAMI, Elisabeth, F-75008 Paris (FR); WEI, Xiaodan, Bridgewater, New Jersey 08807 (US)
(74) Representative: Weickmann & Weickmann PartmbB
(86) International application number: PCT/EP2012/071271
(87) International publication number: WO 2013/060850

(56) References cited:
- EP-A1- 2 329 848
- WO-A1-2012/156299
- U.S. National Institutes of Health: "24-week Treatment With Lixisenatide in Type 2 Diabetes Insufficiently Controlled With Metformin and Insulin Glargine (GetGoal Duo1)", , 8 August 2011 (2011-08-08), XP002689781, Retrieved from the Internet: URL:http://clinicaltrials.gov/archive/NCT0 0975286/2011_08_08 [retrieved on 2013-01-08]
- CAMPAS C ET AL: "AVE-0010 GLP-1 Receptor Agonist Treatment of Diabetes", DRUGS OF THE FUTURE, PROUS SCIENCE, ES, vol. 33, no. 10, 1 October 2008 (2008-10-01), pages 838-840, XP002572655, ISSN: 0377-8282, DOI: 10.1358/DOF.2008.33.10.1265206
- YKI-JARVINEN H ET AL: "Insulin glargine or NPH combined with metformin in type 2 diabetes: the LANMET study", DIABETOLOGIA, SPRINGER, BERLIN, DE, vol. 49, no. 3, 1 March 2006 (2006-03-01), pages 442-451, XP002573816, ISSN: 0012-186X, DOI: 10.1007/S00125-005-0132-0 [retrieved on 2006-02-03]
- TEWS D ET AL: "Enhanced protection against cytokine- and fatty acid-induced apoptosis in pancreatic beta cells by combined treatment with glucagon-like peptide-1 receptor agonists and insulin analogues", HORMONE AND METABOLIC RESEARCH, THIEME-STRATTON, STUTTGART, DE, vol. 40, no. 3, 1 March 2008 (2008-03-01), pages 172-180, XP009130880, ISSN: 0018-5043, DOI: 10.1055/S-2008-1042426
- ARNOLDS SABINE ET AL: "Insulin Glargine (GLAR) plus Metformin (MET): An Efficacious and Safe Regimen That Can Be Combined with Exenatide (EXE) or Sitagliptin (SITA)", DIABETES, AMERICAN DIABETES ASSOCIATION, US, vol. 58, no. Suppl. 1, 1 June 2009 (2009-06-01), page a141, XP009130958, ISSN: 0012-1797

### SEQUENCE LISTING

<110> Sanofi-Aventis Deutschland GmbH
<120> Treatment protocol of Diabetes Type 2
<130> 51977PEP
<140> EP11187169.5
   <141> 2011-10-28
<160> 2
<170> PatentIn version 3.5
<210> 1
   <211> 44
   <212> PRT
   <213> Artificial Sequence
<220>
   <223> AVE0010
<220>
   <221> MOD_RES
   <222> (44)..(44)
   <223> AMIDATION
<400> 1
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys
           35                  40
<210> 2
   <211> 39
   <212> PRT
   <213> Artificial Sequence
<220>
   <223> Exendin-4
<220>
   <221> MOD_RES
   <222> (39)..(39)
   <223> AMIDATION
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35

## Description

Subject of the present invention is a pharmaceutical combination for use in the treatment of a diabetes type 2 patient, wherein the diabetes type 2 is insufficiently controlled by at least one oral antidiabetic drug, said combination comprising (a) desPro³⁶Exendin-4(1-39)-Lys₆-NH₂ or/and a pharmaceutically acceptable salt thereof, (b) insulin glargine or/and pharmaceutically acceptable salt thereof, and (c) metformin or/and a pharmaceutically acceptable salt thereof, wherein the treatment of the diabetes type 2 patient comprises the steps: (i) administration of compounds (b) and (c) for at least 4 weeks, with the proviso that compound (a) is not administered, and (ii) continuing treatment by administration of compounds (a), (b) and (c), wherein at the onset of step (i), the patient has a 2 hours postprandial plasma glucose concentration of at least 12 mmol/L, and a glucose excursion of at least 5 mmol/L, wherein the glucose excursion is the difference of the 2 hours postprandial plasma glucose concentration and plasma glucose concentration 30 minutes prior to a meal test, and wherein the amount of compound (b) to be administered in steps (i) or/and (ii) is adjusted so that a predetermined fasting plasma glucose level or/and a predetermined self monitored plasma glucose level is reached or at least approximated.

In a healthy person the release of insulin by the pancreas is strictly coupled to the concentration of blood glucose. An increased level of blood glucose, as appears after meals, is rapidly counterbalanced by a respective increase in insulin secretion. In fasting condition the plasma insulin level drops to a basal value which is sufficient to ensure the continuous supply of glucose to insulin-sensitive organs and tissues and to keep the hepatic glucose production at a low level at night.

In contrast to diabetes type 1, there is not generally a lack of insulin in diabetes type 2 but in many cases, particularly in progressive cases, the treatment with insulin is regarded as the most suitable therapy, if required in combination with orally administered anti-diabetic drugs.

An increased glucose level in the blood over several years without initial symptoms represents a significant health risk. It could clearly be shown by the large-scale DCCT study in the USA (The Diabetes Control and Complications Trial Research Group (1993) N. Engl. J. Med. 329, 977-986) that chronically increased levels of blood glucose are a main reason for the development of diabetes complications. Examples for diabetes complications are micro and macrovascular damages that possibly manifest themselves in retinopathies, nephropathies or neuropathies and lead to blindness, renal failure and the loss of extremities and are accompanied by an increased risk of cardiovascular diseases. It can thus be concluded that an improved therapy of diabetes primarily has to aim keeping blood glucose in the physiological range as closely as possible.

A particular risk exists for overweight patients suffering from diabetes type 2, e.g. patients with a body mass index (BMI) ≥ 30. In these patients the risks of diabetes overlap with the risks of overweight, leading e.g. to an increase of cardiovascular diseases compared to diabetes type 2 patients being of a normal weight. Thus, it is particularly necessary to treat diabetes in these patients while reducing the overweight.

Metformin is a biguanide hypoglycemic agent used in the treatment of non-insulin-dependent diabetes mellitus (diabetes mellitus type 2) not responding to dietary modification. Metformin improves glycemic control by improving insulin sensitivity and decreasing intestinal absorption of glucose. Metformin is usually administered orally. However, control diabetes mellitus type 2 in obese patients by metformin may be insufficient. Thus, in these patients, additional measures for controlling diabetes mellitus type 2 may be required.

Insulin is a polypeptide having 51 amino acid residues. Insulin consists of the A chain having 21 amino acid residues, and the B chain having 30 amino acid residues. The chains are coupled by 2 disulfide bridges. Insulin formulations have been used for a long time for therapy of diabetes mellitus type 1 and 2. Recently, insulin derivatives and insulin analogues have been used.

The compound desPro³⁶Exendin-4(1-39)-Lys₆-NH₂ (AVE0010, lixisenatide) is a derivative of Exendin-4. Lixisenatide is disclosed as SEQ ID NO:93 in WO 01/04156:
**SEQ ID NO: 1: Lixisenatide (44 AS)** H-G-E-G-T-F-T-S-D-L-S-K-Q-M-E-E-E-A-V-R-L-F-I-E-W-L-K-N-G-G-P-S-S-G-A-P-P-S-K-K-K-K-K-K-NH₂
**SEQ ID NO: 2: Exendin-4 (39 AS)** H-G-E-G-T-F-T-S-D-L-S-K-Q-M-E-E-E-A-V-R-L-F-I-E-W-L-K-N-G-G-P-S-S-G-A-P-P-P-S-NH₂

Exendins are a group of peptides which can lower blood glucose concentration. The Exendin analogue lixisenatide is characterised by C-terminal truncation of the native Exendin-4 sequence. Lixisenatide comprises six C-terminal lysine residues not present in Exendin-4.

The ClinicalTrials.gov homepage discloses a clinical trial protocol NCT 00975286, dated 08 August 2011. The study included patients with type 2 diabetes mellitus, as defined by WHO, having a fasting plasma glucose > or = 7 mmol/L (126 mg/dL) or 2 hours postprandial plasma glucose > or = 11.1 mmol/L (200 mg/dL), diagnosed at least 1 year before the screening visit.

Campas et al., Drugs of the Future, 33:838-840 (2008) is a review article about the pharmacology and clinical trials of lixisenatide.

Yki-Järvinen et al., Diabetologia, 49:442-451 (2006) discloses a study concerning insulin glargine or NPH insulin combined with metformin.

Tews et al., Horm Metab Res 40:172-180 (2008) discloses *in vitro* data of the combination of lixisenatide and insulin glargine.

Arnolds et al., Diabetes 58, Suppl. 1, A141 (2009) discloses a clinical trial of the combination of insulin glargine plus metformin plus exendin-4 or sitagliptin.

EP 2 329 848 discloses the combination of insulin glargine, lixisenatide and metformin.

In the context of the present invention, lixisenatide includes pharmaceutically acceptable salts thereof. The person skilled in the art knows pharmaceutically acceptable salts of lixisenatide. A preferred pharmaceutically acceptable salt of lixisenatide employed in the present invention is acetate.

In the present invention, it has surprisingly been found that the efficacy of a combination of insulin glargine, metformin and lixisenatide can be improved if the treatment starts with administration of a combination of insulin glargine and metformin alone (with optionally a further antidiabetic agent, such as a thiazolidinedione). After such run-in phase, the combination of insulin glargine, metformin and lixisenatide is administered (with optionally a further antidiabetic agent, such as a thiazolidinedione). In the Example of the present invention, during the 12-week run-in phase, insulin glargine resulted in a remarkable reduction in the mean HbA_{1c} value from 8.6% in each group to 7.56% in the lixisenatide group and 7.60% in the placebo group. A further significant reduction of the mean HbA_{1c} value was observed in both treatment groups during the 24-week randomized treatment phase. Surprisingly, the effect was larger in the lixisenatide group (administration of insulin glargine, metformin and lixisenatide) than in the placebo group (administration of insulin glargine, metformin and placebo). In the lixisenatide group, the HbA_{1c} decreased to 6.96% in the lixisenatide group and to 7.30% in the placebo group. Furthermore, by this treatment protocol, the number of patients reaching a HbA_{1c} value <7% is surprisingly larger in the lixisenatide group than in the placebo group. At week 24, 56.3% of patients in the lixisenatide group and 38.5% of patients in the placebo group achieved HbA_{1c} values <7% (p=0.0001).

The daily insulin glargine dose in both groups increased gradually during the 24 weeks test period of the Example of the present invention. Surprisingly, patients in the lixisenatide group showed less increase in daily insulin glargine dose while achieving a greater reduction in HbA_{1c} (LS mean difference versus placebo of 2.24 U, P value = 0.0300). Therefore, by the treatment protocol of diabetes type 2 patients, as described herein, the daily insulin dose can be reduced. This reduction indicates an improved plasma insulin concentration by the treatment protocol as described herein.

A further surprising effect of the treatment protocol, as described herein refers to a significantly improved postprandial glycemic control by treatment with lixisenatide as measured by 2-hour postprandial plasma glucose (PPG) and postprandial glucose excursion. A statistically significant reduction in 2-hour PPG after a standard test-meal from baseline to Week 24 was achieved in the lixisenatide group compared with the placebo group. Correspondingly, a substantial reduction in glucose excursion was observed in the patients treated with lixisenatide compared to those treated with placebo.

Furthermore, treatment with lixisenatide demonstrated a statistically significant improvement in the average of the 7-point self-monitored plasma glucose (SMPG) profile compared with the placebo group.

A first aspect of the present invention is a pharmaceutical combination for use in the treatment of a diabetes type 2 patient, wherein the diabetes type 2 is insufficiently controlled by at least one oral antidiabetic drug, said combination comprising
(a) desPro³⁶Exendin-4(1-39)-Lys₆-NH₂ or/and a pharmaceutically acceptable salt thereof,
(b) insulin glargine or/and pharmaceutically acceptable salt thereof, and
(c) metformin or/and a pharmaceutically acceptable salt thereof,
wherein the treatment of the diabetes type 2 patient comprises the steps:
(i) administration of compounds (b) and (c) for at least 4 weeks, with the proviso that compound (a) is not administered, and
(ii) continuing treatment by administration of compounds (a), (b) and (c),
wherein at the onset of step (i), the patient has a 2 hours postprandial plasma glucose concentration of at least 12 mmol/L, and a glucose excursion of at least 5 mmol/L, wherein the glucose excursion is the difference of the 2 hours postprandial plasma glucose concentration and plasma glucose concentration 30 minutes prior to a meal test, and wherein the amount of compound (b) to be administered in steps (i) or/and (ii) is adjusted so that a predetermined fasting plasma glucose level or/and a predetermined self monitored plasma glucose level is reached or at least approximated.

Metformin is the international nonproprietary name of 1,1-dimethylbiguanide (CAS Number 657-24-9). In the present invention, the term "metformin" includes any pharmaceutically acceptable salt thereof.

In the present invention, metformin may be administered orally. The skilled person knows formulations of metformin suitable for treatment of diabetes type 2 by oral administration. Metformin may be administered to a subject in need thereof, in an amount sufficient to induce a therapeutic effect. Metformin may be administered in a dose of at least 1.0 g/day or at least 1.5 g/day. For oral administration, metformin may be formulated in a solid dosage form, such as a tablet or pill. Metformin may be formulated with suitable pharmaceutically acceptable carriers, adjuvants, or/and auxiliary substances.

Insulin glargine (Lantus) is Gly(A21)-Arg(B31)-Arg(B32)-human insulin. In the present invention, insulin glargine includes pharmaceutically acceptable salts thereof.

Insulin glargine or/and a pharmaceutically acceptable salt thereof may be administered parenterally, e.g. by injection (such as by intramuscular or by subcutaneous injection). The skilled person knows suitable liquid formulations of insulin glargine, including suitable pharmaceutically acceptable carriers, adjuvants or/and auxiliary substances. Suitable injection devices, for instance the so-called "pens" comprising a cartridge comprising the active ingredient, and an injection needle, are known. In the present invention, insulin glargine or/and the pharmaceutically acceptable salt thereof may be administered to a subject in need thereof, in an amount sufficient to induce a therapeutic effect. The insulin glargine or/and a pharmaceutically acceptable salt thereof may be administered, for example, in an amount in the range of 15 to 80 U per dose.

In the present invention, the insulin glargine or/and a pharmaceutically acceptable salt thereof may be administered in a daily dose in the range of 15 to 80 U. Insulin glargine or/and a pharmaceutically acceptable salt thereof may be administered once daily, for example by one injection per day.

In step (i), the compounds (b) and (c) of the pharmaceutical combination of the present invention may be administered for at least 4 weeks, at least 8 weeks, at least 12 weeks, or at least 16 weeks. Preferably, step (i) comprises administration of compounds (b) and (c) for at least about 12 weeks.

Step (i) may be performed for at the maximum about 8 weeks, at the maximum about 12 weeks, at the maximum about 16 weeks, at the maximum about 20 weeks, or at the maximum 24 about weeks. Preferred is a duration of step (i) of about 12 weeks.

Step (i) is performed with the proviso that compound (a) is not administered. As demonstrated by the Example of the present invention, a treatment with a combination of insulin glargine, metformin and lixisenatide can improve postprandial glycemic control, HbA_{1c} value, and the SMPG if the treatment starts with administration of insulin glargine and metformin alone. By this treatment protocol, the dose of insulin glargine can be reduced.

Step (i) or/and step (ii) may comprise the further administration of a thiazolidinedione. Thiazolidinediones (also termed Glitazones) such as pioglitazone are antihyperglycemic agents that reduce insulin resistance by sensitizing muscle, liver and adipose tissue (Dormandy et al., Lancet 2005, 366:1270-89, Yki-Jarvinen, N Engl J Med 2004, 351: 1106-18). In the context of the present invention, "thiazolidinedione", as used herein, includes pharmaceutically acceptable salts thereof. The glitazone may be selected from pioglitazone, troglitazone and rosiglitazone and pharmaceutically acceptable salts thereof. The thiazolidinedione, in particular pioglitazone, may be administered in a dose of at least 10 mg/day, at least 20 mg/day, at least 30 mg/day, or at least 40 mg/day. The maximal daily dose of the thiazolidinedione, in particular pioglitazone, may be 50 mg/day or 60 mg/day. A preferred dosing range is 10 mg/day to 50 mg/day or 30 mg/day to 40 mg/day. A more preferred dose is about 30 mg/day. Rosiglitazone may be administered at a dose of 2 mg/day to 10 mg/day, or 3 mg/day to 8 mg/day. A more preferred dose of rosiglitazone is about 4 mg/day. For oral administration, the thiazolidinedione, in particular pioglitazone, may be formulated in a solid dosage form, such as a tablet or pill. The thiazolidinedione, in particular pioglitazone, may be formulated with suitable pharmaceutically acceptable carriers, adjuvants, or/and auxiliary substances.

The pharmaceutical combination of any one of the preceding claims, wherein administration in steps (i) or/and (ii) is performed on a daily basis. Metformin, lixisenatide and the insulin glargine may be administered within a time interval of 24 h. Metformin, lixisenatide and insulin glargine each may be administered in a once-a-day-dosage. Metformin, lixisenatide and insulin glargine may be administered by different administration routes. Metformin may be administered orally, and lixisenatide and the insulin glargine may be administered parenterally.

In the present invention, desPro³⁶Exendin-4(1-39)-Lys₆-NH₂ or/and a pharmaceutically acceptable salt may be administered in an add-on therapy to administration of insulin glargine and metformin. In the present invention, the terms "add-on", "add-on treatment" and "add-on therapy" relate to treatment of diabetes mellitus type 2 with metformin, lixisenatide and the insulin glargine. The add-on treatment may include the administration of a thiazolidinedione, as described herein.

The subject to be treated by the medicament of the present invention suffering from diabetes type 2 may be a subject suffering from diabetes type 2, wherein diabetes type 2 is not adequately controlled by treatment with at least one oral anti-diabetic drug alone, for example with at least 1.0 g/day metformin or at least 1.5 g/day metformin, for example for 3 months, or with thiazolinedione as described herein, for example for 3 months, or a combination of metformin and a thiazolinedione. In the present invention, a subject the diabetes type 2 of which is not adequately controlled may have a HbA_{1c} value in the range of 7 % to 10% or even larger.

In the pharmaceutical composition of the present invention, the amount of compound (b) to be administered in steps (i) or/and (ii) is adjusted so that a predetermined fasting plasma glucose level or/and a predetermined self monitored plasma glucose level is reached or at least approximated. The amount of compound (b) to be administered in steps (i) or/and (ii) may be adjusted on the basis of daily measurements of plasma glucose concentration. In particular the amount of compound (b) to be administered in steps (i) or/and (ii) may adjusted so that a fasting plasma glucose level of about 4.4 mmol/l to about 5.6 mmol/l or/and a self monitored plasma glucose level (SMPG) of about 8 mmol/l (or about 140 mg/dl) is reached or at least approximated.

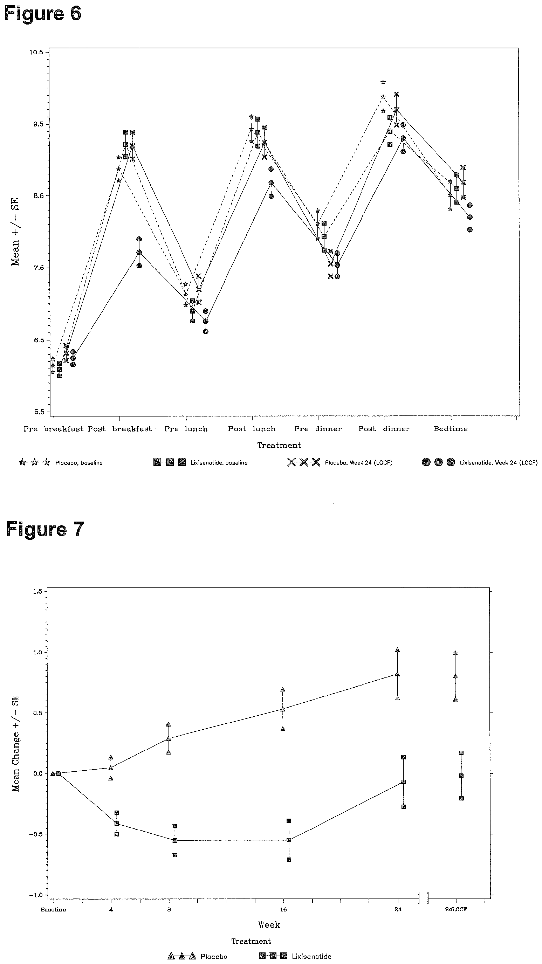

"Self-monitored plasma glucose (SMPG)", as used herein, is in particular the "7-point Self Monitored Plasma Glucose". "7-point Self Monitored Plasma Glucose" in particular refers to the measurement of plasma glucose seven times a day and calculation of the average plasma glucose concentration therefrom. The "7-point Self Monitored Plasma Glucose" value is in particular an average plasma glucose concentration including fasting and postprandial conditions. In particular, measurements of plasma glucose concentration are performed pre-breakfast, post-breakfast, pre-lunch, post-lunch, pre-dinner, post-dinner and at bed-time (see also Figure 6). The treatment by the combination of the present invention, as described herein, can improve the self-monitored plasma glucose.

As demonstrated by the Example disclosed herein, the combination as described herein can be used for improving glycemic control in a diabetes type 2 patient. In particular glycemic control is postprandial glycemic control. More particular postprandial glycemic control is control of postprandial plasma glucose or/and postprandial glucose excursion.

In the present invention, "improvement of glycemic control" or "glycemic control" includes the improvement of glucose tolerance, improvement of postprandial plasma glucose concentration, improvement of postprandial glucose excursion, improvement of fasting plasma glucose concentration, improvement of the HbA_{1c} value or/and improvement of fasting plasma insulin concentration.

In particular, improvement of glucose tolerance includes improvement of the postprandial plasma glucose concentration, improvement of postprandial glucose excursion, or/and improvement of fasting plasma insulin concentration. More particular, improvement of glucose tolerance includes includes improvement of the postprandial plasma glucose concentration.

Improvement of glucose excursion is in particular reduction of glucose excursion. The glucose excursion is at least 5 mmol/L before treatment as described herein.

In particular, improvement of postprandial plasma glucose concentration is reduction of the postprandial plasma glucose concentration. Reduction means in particular that the plasma glucose concentration reaches normoglycemic values or at least approaches these values.

In particular, improvement of fasting plasma glucose concentration is reduction of the fasting plasma glucose concentration. Reduction means in particular that the plasma glucose concentration reaches normoglycemic values or at least approaches these values.

In particular, improvement of the HbA_{1c} value is reduction of the HbA_{1c} value. Reduction of the HbA_{1c} value in particular means that the HbA_{1c} value is reduced below 6.5 % or 7 %, for example after treatment by steps (i) or/and (ii), as described herein, at least two months, at least three months, at least four months, at least five months, at least six months or at least one year.

In particular, improvement of fasting plasma insulin concentration is reduction of fasting plasma insulin concentration. In the Example of the present invention, it was surprisingly found that the dose of insulin glargine could be reduced when administered together with lixisenatide and metformin, as described herein, compared with administration of insulin glargine and metformin alone. The plasma insulin concentration is coupled to the plasma glucose concentration. Under treatment as described herein, in fasting condition the plasma insulin may reach or at least approach values to ensure the continuous supply of glucose to insulin-sensitive organs and tissues or/and to keep the hepatic glucose production at a low level at night. At fasting conditions, the insulin concentration may reach or at least approach values associated with normoglycemia or plasma glucose concentration approaching normoglycemia.

The subject to be treated by the medicament of the present invention suffering from diabetes type 2 may be an obese subject. In the present invention, an obese subject may have a body mass index of at least 30 kg/m².

The subject to be treated by the medicament of the present invention suffering from diabetes type 2 may have a normal body weight. In the present invention, a subject having normal body weight may have a body mass index in the range of 17 kg/m² to 25 kg/m², or 17 kg/m² to <30 kg/m².

The subject to be treated by the medicament of the present invention may be an adult subject. The subject may have an age of at least 18 years of may have an age in the range of 18 to 80 years, of 18 to 50 years, or 40 to 80 years, or 50 to 60 years. The subject may be younger than 50 years.

The subject to be treated by the medicament of the present invention may suffer from diabetes mellitus type 2 for at least 1 year or at least 2 years. In particular, in the subject to be treated, diabetes mellitus type 2 has been diagnosed at least 1 year or at least 2 years before onset of therapy by the medicament of the present invention.

The subject to be treated may have a HbA_{1c} value of at least about 8 % or at least about 7,5% at the onset of step (i). The subject may also have a HbA_{1c} value of about 7 to about 10 % or even larger. The example of the present invention demonstrates that treatment by lixisenatide results in an improved HbA_{1c} value in diabetes type 2 patients.

In yet another aspect of the present invention, the combination as described herein can be used for improving the HbA_{1c} value in a patient suffering from diabetes type 2. Improving the HbA_{1c} value means that the HbA_{1c} value is reduced below 6.5% or 7%, for example after treatment for at least two months, or at least three months.

In the present invention, normoglycemic values are blood glucose concentrations of in particular 60 - 140 mg/dl (corresponding to 3.3 to 7.8 mmol/L). This range refers in particular to blood glucose concentrations under fasting conditions and postprandial conditions.

The subject to be treated has a 2 hours postprandial plasma glucose concentration of at least 12 mmol/L at the onset of step (i). The subject to be treated may also have a 2 hours postprandial plasma glucose concentration of at least 14 mmol/L at the onset of step (i). These plasma glucose concentrations exceed normoglycemic concentrations.

The subject to be treated has a glucose excursion of at least 5 mmol/L at the onset of step (i). In the present invention, the glucose excursion is the difference of the 2 hours postprandial plasma glucose concentration and the plasma glucose concentration 30 minutes prior to a meal test.

"Postprandial" is a term that is well known to a person skilled in the art of diabetology. The term "postprandial" describes in particular the phase after a meal or/and exposure to glucose under experimental conditions. In a healthy person this phase is characterised by an increase and subsequent decrease in blood glucose concentration. The term "postprandial" or "postprandial phase" typically ends up to 2h after a meal or/and exposure to glucose.

The subject to be treated as disclosed herein may have a fasting plasma glucose concentration of at least 8 mmol/L, at least 8,5 mmol/L or at least 9 mmol/L at the onset of step (i). These plasma glucose concentrations exceed normoglycemic concentrations.

The patient to be treated as disclosed herein preferably does not receive an anti-diabetic treatment with an insulin or/and a pharmaceutically acceptable salt thereof at the onset of step (i).

The treatment of the present invention, as described herein, can induce weight loss or/and prevents weight gain in a diabetes type 2 patient. It was surprisingly found in the Example of the present invention that the treatment as described herein can prevent weight gain. During the 24-week treatment period, body weight slightly increased in both groups with a LS mean change of 0.28 kg for the lixisenatide-treated patients and 1.16 kg for the placebo-treated patients. The weight gain was statistically significantly lower in the lixisenatide group than in the placebo group.

The treatment of the present invention, as described herein, can prevent hypoglycaemia in a diabetes type 2 patient. In particular, the pharmaceutical combination is used for the prevention of symptomatic hypoglycaemia or/and severe symptomatic hypoglycaemia in a diabetes mellitus type 2 patient.

In the present invention, hypoglycaemia is a condition wherein a diabetes mellitus type 2 patient experiences a plasma glucose concentration of below 60 mg/dL (or below 3.3 mmol/L), below 50 mg/dL, below 40 mg/dL, or below 36 mg/dL.

By the method of the present invention, hypoglycaemia can be reduced to below 12%, below 11 %, below 10%, below 9%, below 8%, below 7%, below 6% or below 5 % of diabetes type 2 patients receiving the combination of lixisenatide or/and a pharmaceutically acceptable salt thereof, insulin glargine or/and a pharmaceutically acceptable salt thereof and optionally metformin or/and a pharmaceutically acceptable salt thereof, as described herein.

In the present invention, "symptomatic hypoglycaemia" is a condition associated with a clinical symptom that results from the hypoglycaemia, wherein the plasma glucose concentration is below 60 mg/dL (or below 3.3 mmol/L), below 50 mg/dL, or below 40 mg/dL. A clinical symptoms can be, for example, sweating, palpitations, hunger, restlessness, anxiety, fatigue, irritability, headache, loss of concentration, somnolence, psychiatric disorders, visual disorders, transient sensory defects, transient motor defects, confusion, convulsions, and coma. In the present invention, one or more clinical symptoms of symptomatic hypoglycaemia, as indicated herein, can be selected.

Symptomatic hypoglycaemia may be associated with prompt recovery after oral carbohydrate administration.

In the present invention, "severe symptomatic hypoglycaemia" is a condition with a clinical symptom, as indicated herein, that results from hypoglycaemia, wherein the plasma glucose concentration is below 36 mg/dL (or below 2.0 mmol/L). Severe symptomatic hypoglycaemia can be associated with acute neurological impairment resulting from the hypoglycaemic event. In a severe symptomatic hypoglycaemia, the patient may require the assistance of another person, if, for example, the patient could not treat or help him/herself due to the acute neurological impairment. The definition of severe symptomatic hypoglycaemia may include all episodes in which neurological impairment is severe enough to prevent self-treatment and which were thus thought to place patients at risk for injury to themselves or others. The acute neurological impairment may be at least one selected from somnolence, psychiatric disorders, visual disorders, transient sensory defects, transient motor defects, confusion, convulsions, and coma.

Severe symptomatic hypoglycaemia may be associated with prompt recovery after oral carbohydrate, intravenous glucose, or/and glucagon administration. In the present invention, desPro³⁶Exendin-4(1-39)-Lys₆-NH₂ or/and the pharmaceutically acceptable salt thereof may be administered to a subject in need thereof, in an amount sufficient to induce a therapeutic effect.

In the present invention, desPro³⁶Exendin-4(1-39)-Lys₆-NH₂ or/and the pharmaceutically acceptable salt thereof may be formulated with suitable pharmaceutically acceptable carriers, adjuvants, or/and auxiliary substances.

The compound desPro³⁶Exendin-4(1-39)-Lys₆-NH₂ or/and a pharmaceutically acceptable salt thereof may be administered parenterally, e.g. by injection (such as by intramuscular or by subcutaneous injection). Suitable injection devices, for instance the so-called "pens" comprising a cartridge comprising the active ingredient, and an injection needle, are known. The compound desPro³⁶Exendin-4(1-39)-Lys₆-NH₂ or/and a pharmaceutically acceptable salt thereof may be administered in a suitable amount, for instance in an amount in the range of 10 to 15 µg per dose or 15 to 20 µg per dose.

In the present invention, desPro³⁶Exendin-4(1-39)-Lys₆-NH₂ or/and a pharmaceutically acceptable salt thereof may be administered in a daily dose in the range of 10 to 20 µg, in the range of 10 to 15 µg, or in the range of 15 to 20 µg. DesPro³⁶Exendin-4(1-39)-Lys₆-NH₂ or/and a pharmaceutically acceptable salt thereof may be administered by one injection per day.

In the present invention, desPro³⁶Exendin-4(1-39)-Lys₆-NH₂ or/and a pharmaceutically acceptable salt thereof may be provided in a liquid composition. The skilled person knows liquid compositions of lixisenatide suitable for parenteral administration. A liquid composition of the present invention may have an acidic or a physiologic pH. An acidic pH preferably is in the range of pH 1 - 6.8, pH 3.5 - 6.8, or pH 3.5 - 5. A physiologic pH preferably is in the range of pH 2.5 - 8.5, pH 4.0 - 8.5, or pH 6.0 - 8.5. The pH may be adjusted by a pharmaceutically acceptable diluted acid (typically HCl) or pharmaceutically acceptable diluted base (typically NaOH).

The liquid composition comprising desPro³⁶Exendin-4(1-39)-Lys₆-NH₂ or/and a pharmaceutically acceptable salt thereof may comprise a suitable preservative. A suitable preservative may be selected from phenol, m-cresol, benzyl alcohol and p-hydroxybenzoic acid ester. A preferred preservative is m-cresol.

The liquid composition comprising desPro³⁶Exendin-4(1-39)-Lys₆-NH₂ or/and a pharmaceutically acceptable salt thereof may comprise a tonicity agent. A suitable tonicity agent may be selected from glycerol, lactose, sorbitol, mannitol, glucose, NaCl, calcium or magnesium containing compounds such as CaCl₂. The concentration of glycerol, lactose, sorbitol, mannitol and glucose may be in the range of 100 - 250 mM. The concentration of NaCl may be up to 150 mM. A preferred tonicity agent is glycerol.

The liquid composition comprising desPro³⁶Exendin-4(1-39)-Lys₆-NH₂ or/and a pharmaceutically acceptable salt thereof may comprise methionine from 0.5 µg/mL to 20 µg/mL, preferably from 1 µg /ml to 5 µg/ml. Preferably, the liquid composition comprises L-methionine.

Also described herein is a method for treatment of a diabetes type 2 patient, wherein the diabetes type 2 is insufficiently controlled by at least one oral antidiabetic drug, wherein the method comprises the administration of a combination, said combination comprising
(a) desPro³⁶Exendin-4(1-39)-Lys₆-NH₂ or/and a pharmaceutically acceptable salt thereof,
(b) insulin glargine or/and pharmaceutically acceptable salt thereof, and
(c) metformin or/and a pharmaceutically acceptable salt thereof,
wherein the administration of the combination comprises the steps:
(i) administration of compounds (b) and (c) for at least 4 weeks, and
(ii) continuing treatment by administration of compounds (a), (b) and (c),
wherein the amount of compound (b) to be administered in steps (i) or/and (ii) is adjusted so that a predetermined fasting plasma glucose level or/and a predetermined self monitored plasma glucose level is reached or at least approximated.

In particular, in the method as described herein, a combination as described herein can be administered. More particular, the compounds (a),
(b) and (c) are compounds as defined herein. In particular, the patient is a patient as defined herein. Further, steps (i) and (ii) are performed in particular as defined herein. Furthermore, adjustment of the compound (b) to be administered in steps (i) and (ii) is in particular performed as disclosed herein,

Also described herein is the use of a combination comprising
(a) desPro³⁶Exendin-4(1-39)-Lys₆-NH₂ or/and a pharmaceutically acceptable salt thereof,
(b) insulin glargine or/and pharmaceutically acceptable salt thereof, and
(c) metformin or/and a pharmaceutically acceptable salt thereof,
for the manufacture of a medicament for the treatment of a diabetes type 2 patient, wherein the diabetes type 2 is insufficiently controlled by at least one oral antidiabetic drug, and wherein the treatment by the combination comprises the steps:
(i) administration of compounds (b) and (c) for at least 4 weeks, and
(ii) continuing treatment by administration of compounds (a), (b) and (c),
wherein the amount of compound (b) to be administered in steps (i) or/and (ii) is adjusted so that a predetermined fasting plasma glucose level or/and a predetermined self monitored plasma glucose level is reached or at least approximated.

In particular, in the use as described herein, a combination as described herein can be administered. More particular, the compounds (a), (b) and (c) are compounds as defined herein. In particular, a patient as defined herein can be treated by the medicament. Further, steps (i) and (ii) are performed in particular as defined herein. Furthermore, adjustment of the compound (b) to be administered in steps (i) and (ii) is in particular performed as disclosed herein,

The invention is further illustrated by the following example and figures.

### Figure legends

**Figure 1****:** Study design. For the run-in phase a visit window of ±3 days is acceptable using the date of visit 2 as reference. During the randomized double-blind treatment period a visit window of ±3 days up to visit 15 (week 2) and ±5 days after visit 15 is acceptable, using the day of visit 13 as reference. A visit window of -1 day or + 3 days is acceptable for the post-treatment follow-up visit using the day of visit 22 as reference. * Volume matched placebo.
**Figure 2****:** Kaplan-Meier plot of time to treatment discontinuation due to any reason - Randomized population.
**Figure 3****:** Plot of mean change in HbA_{1c} (%) from baseline by visit - mITT population. LOCF = Last observation carried forward. Note: The plot excluded measurements obtained after the introduction of rescue medication and/or after the treatment cessation plus 14 days.
**Figure 4****:** Plot of mean HbA_{1c} (%) by visit - mITT population. LOCF = Last observation carried forward. Note: The plot excluded measurements obtained after the introduction of rescue medication and/or after the treatment cessation plus 14 days.
**Figure 5****:** Plot of mean change in average 7-point Self Monitored Plasma Glucose (SMPG) (mmol/L) from baseline by visit - mITT population. LOCF = Last observation carried forward. Note: The plot excluded measurements obtained after the introduction of rescue medication and/or after the treatment cessation.
**Figure 6****:** Plot of mean 7-point Self Monitored Plasma Glucose (SMPG) (mmol/L) by each time point, at baseline and Week 24 (LOCF) - mITT population. Note: The baseline value is defined as the last available value prior to the first injection of double-blind investigational product. The plot excluded measurements obtained after the introduction of rescue medication and/or after the treatment cessation.
**Figure 7****:** Plot of mean change in body weight (kg) from baseline by visit - mITT population. LOCF = Last observation carried forward. Note: The plot excluded measurements obtained after the introduction of rescue medication and/or after the treatment cessation plus 3 days.
**Figure 8****:** Plot of mean change in insulin glargine dose (U) from baseline by visit- mITT population. LOCF = Last observation carried forward. Bas. = Baseline. Note: The plot excluded measurements obtained after the introduction of rescue medication and/or after the treatment cessation.
**Figure 9****:** Plot of mean change in fasting plasma glucose (mmol/L) from baseline by visit - mITT population. LOCF = Last observation carried forward. Note: The plot excluded measurements obtained after the introduction of rescue medication and/or after the treatment cessation plus 1 day.

### EXAMPLE

### SUMMARY

The Example refers to a randomized, double-blind, placebo-controlled, 2-arm, parallel-group, multinational study assessing the efficacy and safety of lixisenatide in comparison to placebo as an add-on treatment to insulin glargine and metformin in combination with or without TZDs in patients with type 2 diabetes. The approximately maximum study duration per patient was 39 weeks [up-to 14-week screening period (including an up to 2-week screening phase and a 12-week run-in phase) + a 24-week double-blind, placebo-controlled treatment period + a 3-day follow-up period]. The study was conducted in 140 centers in 25 countries. The primary objective of this study was to assess the effects on glycemic control of lixisenatide in comparison to placebo as an add-on treatment to insulin glargine and metformin in terms of HbA1c change over a period of 24 weeks.

A total of 446 patients were randomized to one of the two treatment groups (223 in the lixisenatide group and 223 in the placebo group) and all of the randomized patients were exposed to the investigational product (IP). Demographics and baseline characteristics were generally similar across the treatment groups. No patient was excluded from the mITT population for efficacy analyses. During the study treatment period, 29 (13.0%) lixisenatide-treated patients prematurely discontinued the IP, while 12 (5.4%) placebo-treated patients discontinued the IP. For both treatment groups, the main reason for treatment discontinuation was "adverse event" (8.5% for lixisenatide versus 4.0% for placebo) followed by "other reasons" (3.6% for lixisenatide versus 1.3% for placebo). Of note, GI related AEs were the major TEAEs leading to IP discontinuation for lixisenatide (10 patients [4.5%]).

HbAlc decreased in both treatment groups from a value of 7.56% at baseline to 6.96% at week 24 (LOCF) in the lixisenatide group and from 7.60% to 7.30% in the placebo group. The HblAc decrease for lixisenatide was significantly greater compared to placebo: the least squared (LS) mean changes from baseline to Week 24 were -0.71% and -0.40%, respectively, and LS mean difference vs. placebo was -0.32%, with a p-value < 0.0001. This is worth noting that, per protocol, insulin dose adjustments to maintain fasting plasma glucose at target were allowed in both treatment groups throughout the study.

A total of 121 patients (56.3%) in the lixisenatide group achieved HbA_{1c} <7% at Week 24 compared to 85 patients (38.5%) in the placebo group, and 69 (32.1%) lixisenatide-treated patients had HBA_{1c} ≤ 6.5% compared to 36 (16.3%) of placebo-treated patients. The HbA_{1c} responder analysis (HbA_{1c} ≤6.5 or <7% at Week 24) using Cochran-Mantel-Haenszel (CMH) method showed a significant treatment difference between lixisenatide and placebo at Week 24 (p-value<0.0001 and p-value =0.0001, respectively).

Treatment with lixisenatide significantly improved postprandial glycemic control as measured by 2-hour postprandial plasma glucose (PPG) and postprandial glucose excursion. A statistically significant reduction in 2-hour PPG after a standard test-meal from baseline to Week 24 was achieved in the lixisenatide group compared with the placebo group, with a LS mean difference of -3.16 mmol/L (p-value <.0001). Correspondingly, a substantial reduction in glucose excursion was observed in the patients treated with lixisenatide compared to those treated with placebo (LS mean difference = -3.09 mmol/L, 95% CI = -3.842 to -2.331).

Furthermore, treatment with lixisenatide demonstrated a statistically significant improvement in the average of the 7-point self-monitored plasma glucose (SMPG) profile (LS mean difference of -0.39 mmol/L; p-value 0.0071) compared with the placebo group.

A statistically significant less weight gain was observed in the lixisenatide group than in the placebo group (LS mean body weight change from baseline to Week 24 was 0.28 kg for the lixisenatide-treated patients and 1.16 kg for the placebo-treated patients; LS mean difference versus placebo = -0.89 kg, p-value = 0.0012)

Over the 24 week on-treatment period, in both groups, the daily insulin dose increased gradually which was permitted by the protocol to maintain FPGs between 100 and 80 mg/d (5.6 and 4.4 mmmol/L)(LS mean change from baseline was 3.10 U in the lixisenatide group and 5.34 in the placebo group). However, patients in the lixisenatide group showed a significantly less increase in daily insulin glargine dose while achieving a greater reduction in HbA1c (LS mean difference versus placebo = -2.24 U; p-value =0.0300).

For fasting plasma glucose, no statistically significant difference was observed between treatment groups (LS mean difference versus placebo = -0.12 mmol/L; p-value =0.5142). A total of 2 patients (1 [0.4%] in each group) received a rescue therapy.

Lixisenatide was well tolerated. The safety profile in the lixisenatide group was generally comparable to the placebo group although the number of the patients with treatment emergent adverse events (TEAEs) was slightly higher in the lixisenatide group [178 (79.8%)] than that in the placebo group [152 (68.2%)]. This disproportion in the number of patients with TEAEs was primarily driven by the GI related AEs (39.9% for lixisenatide versus 16.1% for placebo).

Two patients in the placebo group and no patient in lixisenatide group had TEAEs leading to death.

The number of patients with serious TEAEs was 17 (7.6%) in the lixisenatide group and 10 (4.5%) in the placebo group without a notable increased occurrence in any specific System Organ Classes (SOC).

Fifty (22.4%) lixisenatide-treated patients and 30 (13.5%) patients in the placebo group reported symptomatic hypoglycemic events as defined in the protocol during the on-treatment period. One patient in the lixisenatide group (0.4%) and no patient in the placebo group experienced one event of severe symptomatic hypoglycemia per the protocol definition.

Aside from hypoglycemia, the most frequently reported TEAE was nausea (27.4%) for the lixisenatide group and influenza (6.3%) for the placebo group.

A total of 4 patients (3 [1.3%] lixisenatide-treated patients and 1 [0.4%] placebo-treated patients) reported 4 TEAEs adjudicated as an allergic reaction by the Allergic Reaction Assessment Committee (ARAC), and three of these events (2 events of urticaria in the lixisenatide group and 1 event of dermatitis in the placebo group) were adjudicated as possibly related to the IP. Fifteen patients (6.7%) in the lixisenatide group and 5 patients (2.2%) in the placebo group experienced injection site reaction AEs.

In the placebo group, 1 patient reported 1 TEAE of suspected pancreatitis and 2 patients reported 2 TEAEs of blood calcitonin increase, whereas no patients in the lixisenatide group reported such TEAEs.

### 1 OBJECTIVES

### 1.1 PRIMARY OBJECTIVE

The primary objective of this study was to assess the effects on glycemic control of lixisenatide in comparison to placebo as an add-on treatment to insulin glargine and metformin in terms of HbA1c change over a period of 24 weeks.

### 1.2 SECONDARY OBJECTIVE(S)

The secondary objectives were to:
- Assess the effects of lixisenatide on
   - The percentage of patients reaching HbA1c <7% and ≤6.5%
   - Plasma glucose (fasting, post-prandial during a standardized meal challenge test, 7-point self monitored profiles)
   - Body weight
   - Insulin glargine doses
- Evaluate lixisenatide safety and tolerability (including anti-lixisenatide antibody assessment) as add on treatment to insulin glargine and metformin
- Assess the impact of lixisenatide on treatment satisfaction using the Diabetes Treatment Satisfaction Questionnaire (state) (DTSQs) in participating countries where it was validated.

### 2 TRIAL DESIGN

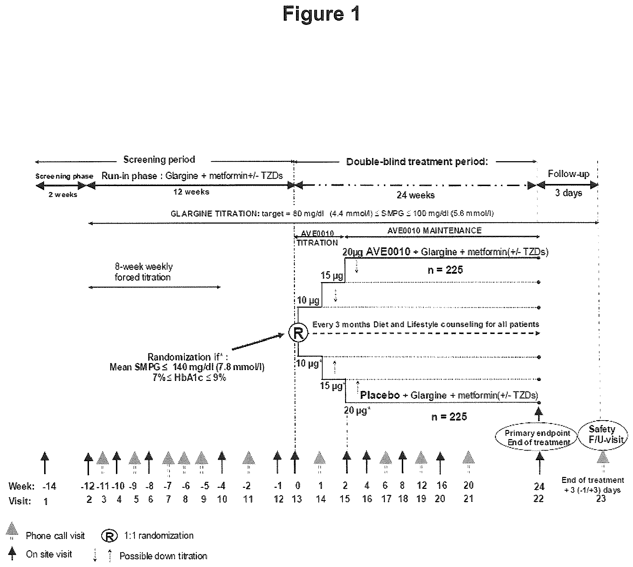

This was a multicenter, multi-national, double-blind, 1:1 randomized, placebo-controlled, 2-arm parallel-group Phase 3 study. The study was double-blind with regard to active and placebo treatments. The study drug volume (ie, dose of active drug or matching placebo) was not blinded. The study design is illustrated by Figure 1.

Patients were stratified by glycosylated hemoglobin A1c (HbAlc) values collected at Visit 12, which was scheduled one week prior to the end of the run-in phase (<8%, ≥ 8%), and Thiazolidinediones (TZD) use (yes, no).

TZDs were the only allowed concomitant additional diabetes treatment to insulin glargine and metformin that could be continued during the study. At the end of the run-in phase, eligible patients were centrally randomized via an interactive response system (IVRS) in a 1:1 ratio to either lixisenatide or placebo. Forced randomization was not allowed.

The study consisted of 3 periods: (1) an up to 14-week screening period, which included an up to 2-week screening phase and a 12-week run-in phase with the introduction and titration of insulin glargine on top of metformin +/- TZDs; patients started insulin glargine once daily and titrated the insulin dose by a treat-to-target regimen to reach a glycemic target of FPG 100 -80 mg/dl (5.6-4.4 mmol/L) during run-in. (2) a 24-week double-blind randomized treatment period for those patients whose HbA1c (centralized assay) was ≥7% and ≤9% and whose mean fasting Self Monitored Plasma Glucose (SMPG) during the 7 days prior to Visit 12 was ≤140 mg/dl (7.8 mmol/l); and (3) a follow-up period with a safety telephone visit (last study visit) 3 (-1/+3) days after the end of treatment visit.

Patients who prematurely discontinued the study treatment were continued in the study up to the scheduled date of study completion. They were followed up according to the study procedures specified in the protocol (except the meal challenge test and treatment satisfaction assessment).

### 3 PRIMARY AND KEY SECONDARY ENDPOINTS

### 3.1 PRIMARY ENDPOINT

The primary efficacy variable was the absolute change in HbA_{1c} from baseline to Week 24, which is defined as: HbA_{1c} value at Week 24 - HbA_{1c} at baseline.

If a patient permanently discontinued the double-blind treatment or received rescue therapy during the 24-week double-blind treatment period or did not have an HbA_{1c} value at Week 24, the last post-baseline HbA_{1c} measurement during the on-treatment period was used as the HbA_{1c} value at Week 24 (last observation carried forward [LOCF] procedure).

### 3.2 SECONDARY ENDPOINTS

### 3.2.1 Efficacy endpoints

For secondary efficacy variables, the same procedure for handling missing assessments/early discontinuation was applied as for the primary efficacy variable.

### Continuous variables

- Change in 2-hour PPG (mmol/L) after the standardized test meal from baseline to Week 24;
- Change in blood glucose excursion (2-hour PPG - plasma glucose 30 minutes prior to the meal test before IP administration) (mmol/L) after the standardized meal challenge test from baseline to Week 24;
- Change in the 7-point SMPG profiles (mmol/L) (ie, the daily average and each timepoint of the 7 points) from baseline to Week 24;
- Change in FPG (mmol/L) from baseline to Week 24;
- Change in body weight (kg) from baseline to Week 24;
- Change in average daily insulin glargine dose (U) from baseline to Week 24;
- Change in treatment satisfaction score (sum of items 1, 4,5,6,7 and 8 from DTSQs) from baseline to Week 24;
- Change in each individual item (Items 1 through 8) from the DTSQs from baseline to Week 24.

### Categorical variables

- Percentage of patients with HbA1c <7% at Week 24;
- Percentage of patients with HbA1c ≤6.5% at Week 24; and
- Percentage of patients requiring rescue therapy during the on-treatment period.

### 3.2.2 Safety endpoints

The safety analysis was based on the reported TEAEs and other safety information including symptomatic hypoglycemia and severe symptomatic hypoglycemia, local tolerability at injection site, allergic events (as adjudicated by ARAC), suspected pancreatitis, increased calcitonin, vital signs, 12-lead ECG and laboratory tests.

According to the protocol:
- Symptomatic hypoglycemia was defined as an event with clinical symptoms with an accompanying plasma glucose <60 mg/dL (3.3 mmol/L) or associated with prompt recovery after oral carbohydrate administration if no plasma glucose measurement was available
- Severe symptomatic hypoglycemia was defined as an event with clinical symptoms in which the patient required the assistance of another person, because the patient could not treat him/herself due to acute neurological impairment directly resulting from the hypoglycemic event, and one of the following:
   - The event was associated with a plasma glucose level below 36 mg/dL (2.0 mmol/L).
   - If no plasma glucose measurement was available, then the event was associated with prompt recovery after oral carbohydrate, intravenous glucose, or glucagon administration

Major cardiovascular events were also collected and sent for adjudication by a Cardiovascular Adjudication Committee (CAC). The adjudicated and confirmed events by CAC from this study and other lixisenatide phase 3 studies will be pooled as necessary for analyses and summarized in a separate report based on the statistical analysis plan for the overall cardiovascular assessment of lixisenatide. The KRM/CSR will not present the summary of the adjudicated and confirmed CV events from this study.

### 4 SAMPLE SIZE CALCULATION ASSUMPTIONS

The sample size/power calculations were performed based on the primary variable, change from baseline to Week 24 in HbA1c.

A sample size of 450 patients (225 patients per group) was expected to provide a power of 98% to detect differences of 0.5% and 90% to detect differences of 0.4% in the change from baseline to Week 24 in HbA1c between lixisenatide and placebo assuming the common standard deviation was 1.3% with a 2-sided test at the 5% significance level.

### 5 STATISTICAL METHODS

### 5.1 ANALYSIS POPULATIONS

The mITT population consisted of all patients who were randomized, received at least one dose of double-blind Investigational Product (IP), and had both a baseline assessment and at least one post-baseline assessment of any primary or secondary efficacy variables, irrespective of compliance with the study protocol and procedures.

The safety population was the total treated population defined as all patients randomized (via the central randomization system according to the protocol) and exposed to at least one dose of the double-blind IP, regardless of the amount of treatment administered.

### 5.2 PRIMARY EFFICACY ANALYSIS

The primary efficacy variable (change in HbA_{1c} from baseline to Week 24) was analyzed using an analysis of covariance (ANCOVA) model with treatment groups (lixisenatide or placebo), randomization strata of Visit 12 HbA_{1c} (<8.0, ≥8.0%), randomization strata of TZDs use (Yes, No), and country as fixed effects and baseline HbA1c value as a covariate. Difference between lixisenatide and placebo and two-sided 95% confidence interval as wells as p-value were estimated within the framework of ANCOVA.

The baseline for the primary efficacy variable was the last available value prior to the first injection of double blind IP (lixisenatide or placebo).

The LOCF procedure was used by taking the last available post-baseline on-treatment HbA_{1c} measurement (before the initiation of the new medication in the event of rescue therapy) as the HbA_{1c} value at Week 24.

The primary analysis of the primary efficacy variable was performed based on the mITT population and the measurements obtained during the on-treatment period for efficacy variables. The on-treatment period for the efficacy variables was defined as the time from the first dose of the double-blind IP up to 14 days for HbA1c; 1 day for FPG by the central laboratory; 0 day for the meal challenge parameters, 7-point SMPG, and insulin glargine; and 3 days for body weight and the treatment satisfaction score after the last dose of the double-blind IP or up to the introduction of rescue therapy, whichever was the earliest.

### 5.3 SECONDARY EFFICACY ANALYSIS

Once the primary variable was statistically significant at α=0.05, the testing procedure was performed to test the following secondary efficacy variables by the following prioritized order. The tests stop as soon as an endpoint was found not statistically significant at α=0.05.
1. Change in 2-hour postprandial plasma glucose (mmol/L) after a standardized meal test from baseline to Week 24,
2. Change in the daily average of the 7-point SMPG from baseline to Week 24,
3. Change in body weight (kg) from baseline to Week 24,
4. Change in average daily insulin glargine dose (U) from baseline to Week 24,
5. Change in FPG (mmol/L) from baseline to Week 24,
6. Percentage of patients requiring rescue therapy during the treatment period.

No multiplicity adjustment was made on the other secondary efficacy variables, which are not mentioned above.

The baseline for secondary efficacy variables was the last available value prior to the first injection of double blind IP (lixisenatide or placebo) except for insulin glargine dose (average daily dose at baseline was the average daily dose for the week prior to Visit 12 which took place at Week -1).

All continuous secondary efficacy variables at Week 24 were analyzed using a similar ANCOVA model as described above for the primary analysis of the primary efficacy endpoint. The estimates of the treatment mean difference between lixisenatide and placebo and two-sided 95% confidence intervals were provided.

The following categorical secondary efficacy variables at Week 24 were analyzed using a Cochran-Mantel-Haenszel (CMH) method stratified on randomization strata (Visit 12 HbA_{1c} [<8.0, ≥8%] and TZDs use [Yes, No]):
- Percentage of patients with HbA_{1c} <7.0% at Week 24,
- Percentage of patients with HbA_{1c} ≤6.5% at Week 24,
- Percentage of patients requiring rescue therapy during the treatment period.

Number and percentage of patients with ≥5% weight loss from baseline at Week 24 are presented by treatment groups.

### 5.4 SAFETY ANALYSIS

The safety analyses were primarily based on the on-treatment period. The **on-treatment period** for safety analysis was defined as the time from the first dose of double-blind IP up to 3 days after the last dose of double-blind IP, regardless of the introduction of rescue status. The 3-day interval was chosen based on the half-life of the double-blind IP (approximately 5 times the half-life).

The summary of safety results (descriptive statistics or frequency tables) is presented by treatment groups.

### 6 RESULTS

### 6.1 STUDY PATIENTS

### 6.1.1 Patient accountability

A total of 1470 patients were screened from 140 centers in 25 countries (Argentina, Brazil, Canada, Chile, Taiwan, Colombia, Czech Republic, Denmark, Estonia, France, Germany, Hungary, India, Israel, Italy, Malaysia, Mexico, Netherlands, Poland, Romania, Russian Federation, South Africa, Sweden, Ukraine and United States of America). Of the 1470 screened patients, 898 entered the 12 weeks of run-in phase. The main reason for screening failure was HbA1c value at screening visit out of the protocol defined range (354 [24.1%] out of 1470 screened patients).

A total of 446 patients were randomized to one of the two treatment groups. The main reason for run-in failure was HbA1c value at Visit 12 (Week -1) was out of the protocol defined range (304 [20.7%] out of 1470 screened patients). All 446 randomized patients were exposed to the IP. No patients were excluded from mITT population for efficacy analyses. Table 1 provides the number of patients included in each analysis population.

**Table 1 Analysis populations - Randomized population**

| | **Placebo (N=223)** | **Lixisenatide (N=223)** | **All (N=446)** |
|---|---|---|---|
| Randomized population | 223 (100%) | 223 (100%) | 446 (100%) |
| | | | |
| Efficacy population | | | |
| Modified Intent-to-Treat (mITT) | 223 (100%) | 223 (100%) | 446 (100%) |
| | | | |
| Safety population | 223 | 223 | 446 |

| | | | |
|---|---|---|---|
| Note: The safety population patients are tabulated according to treatment actually received (as treated). For the efficacy population, patients are tabulated according to their randomized treatment (as randomized). | | | |

### 6.1.2 Study disposition

Table 2 provides the summary of patient disposition for each treatment group.

During the 24-week study treatment period, 29 (13.0%) lixisenatide-treated patients prematurely discontinued the IP, while 12 (5.4%) placebo-treated patients discontinued the IP. For both treatment groups, the main reason for treatment discontinuation was "adverse event" (19 patients [8.5%] for lixisenatide and 9 patients [4.0%] for placebo). Of note, GI related AE was the major TEAEs leading to IP discontinuation for lixisenatide (10 patients [4.5%]). The second most common reason for treatment discontinuation was "other reasons" (8 patients [3.6%] for lixisenatide versus 3 patients [1.3%] for placebo), mostly being personal reasons but also including withdrawals of the non-eligible patients randomized by mistake (3 patients in lixisenatide group and 1 patient in placebo group). Three patients died during the study: two in the placebo group died of a TEAE and one in the lixisenatide group died of a non-TEAE during the post-treatment period.

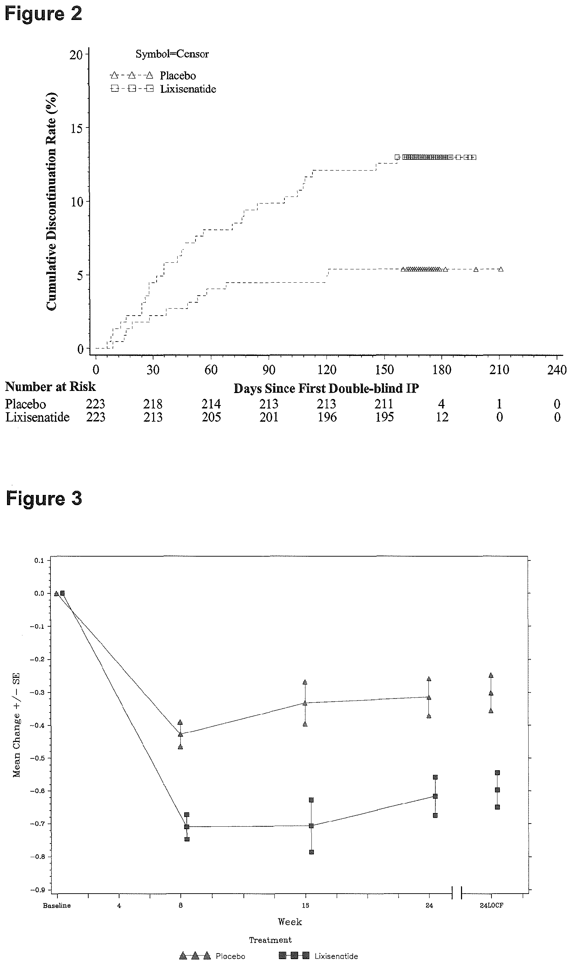

The time-to-onset of treatment discontinuation due to any reason for the 24-week treatment period is depicted in Figure 2. A higher discontinuation rate was observed for the lixisenatide group.

**Table 2 Patient disposition - Randomized population**

| | **Placebo (N=223)** | **Lixisenatide (N=223)** |
|---|---|---|
| Randomized and treated | 223 (100%) | 223 (100%) |
| Did not complete double-blind study treatment | 12 (5.4%) | 29 (13.0%) |
| | | |
| Subject's request for treatment discontinuation | 8 (3.6%) | 17 (7.6%) |
| | | |
| Reason for study treatment discontinuation | 12 (5.4%) | 29 (13.0%) |
| Adverse event | 9 (4.0%) | 19 (8.5%) |
| Lack of efficacy | 0 | 0 |
| Poor compliance to protocol | 0 | 2 (0.9%) |
| Lost to follow-up | 0 | 0 |
| Other reasons | 3 (1.3%) | 8 (3.6%) |
| | | |
| Status at last study contact | 223 (100%) | 223 (100%) |
| Alive | 221 (99.1%) | 222 (99.6%) |
| Dead | 2 (0.9%) | 1 (0.4%) |
| Lost to follow-up | 0 | 0 |

| | | |
|---|---|---|
| Note: Percentages are calculated using the number of randomized patients as denominator. | | |

### 6.1.3 Demographics and baseline characteristics

The demographic and patient baseline characteristics were generally similar between treatment groups for the safety population (Table 3). The median age of the study population was 57.0 years. The majority of the patients were Caucasian (74.4%).

**Table 3 Demographics and patient characteristics at screening or baseline - Safety population**

| | **Placebo (N=223)** | **Lixisenatide (N=223)** | **All (N=446)** |
|---|---|---|---|
| Age (years) | | | |
| Number | 223 | 223 | 446 |
| Mean (SD) | 56.1 (10.2) | 56.4 (9.7) | 56.2 (9.9) |
| Median | 57.0 | 56.0 | 57.0 |
| Min : Max | 25 : 81 | 33 : 80 | 25 : 81 |
| | | | |
| Age group (years) [n (%)] | | | |
| Number | 223 | 223 | 446 |
| < 50 | 56 (25.1%) | 53 (23.8%) | 109 (24.4%) |
| ≥ 50 to < 65 | 124 (55.6%) | 123 (55.2%) | 247 (55.4%) |
| ≥ 65 to < 75 | 38 (17.0%) | 41 (18.4%) | 79 (17.7%) |
| ≥ 75 | 5 (2.2%) | 6 (2.7%) | 11 (2.5%) |
| | | | |
| Gender [n (%)] | | | |
| Number | 223 | 223 | 446 |
| Male | 113 (50.7%) | 109 (48.9%) | 222 (49.8%) |
| Female | 110 (49.3%) | 114 (51.1%) | 224 (50.2%) |
| Race [n (%)] | | | |
| Number | 223 | 223 | 446 |
| Caucasian/White | 167 (74.9%) | 165 (74.0%) | 332 (74.4%) |
| Black | 11 (4.9%) | 9 (4.0%) | 20 (4.5%) |
| Asian/Oriental | 43 (19.3%) | 44 (19.7%) | 87 (19.5%) |
| Other | 2 (0.9%) | 5 (2.2%) | 7 (1.6%) |
| | | | |
| Ethnicity [n (%)] | | | |
| Number | 223 | 223 | 446 |
| Hispanic | 49 (22.0%) | 52 (23.3%) | 101 (22.6%) |
| Not Hispanic | 174 (78.0%) | 171 (76.7%) | 345 (77.4%) |
| | | | |
| HbA1c (%) at Visit 12 (Week -1) | | | |
| Number | 221 | 219 | 440 |
| Mean (SD) | 7.70 (0.54) | 7.69 (0.52) | 7.70 (0.53) |
| Median | 7.60 | 7.60 | 7.60 |
| Min : Max | 7.0 : 9.0 | 7.0 : 9.0 | 7.0 : 9.0 |
| | | | |
| Randomization strata of Visit 12 (Week-1) HbA1c (%) [n(%)] | | | |
| Number | 223 | 223 | 446 |
| <8 | 156 (70.0%) | 157 (70.4%) | 313 (70.2%) |
| ≥8 | 67 (30.0%) | 66 (29.6%) | 133 (29.8%) |
| | | | |
| Randomization strata of TZDs use [n (%)] | | | |
| Number | 223 | 223 | 446 |
| Yes | 25 (11.2%) | 24 (10.8%) | 49 (11.0%) |
| No | 198 (88.8%) | 199 (89.2%) | 397 (89.0%) |
| | | | |
| Baseline BMI (kg/m²) | | | |
| Number | 223 | 223 | 446 |
| Mean (SD) | 31.65 (6.01) | 31.99 (6.63) | 31.82 (6.32) |
| Median | 30.74 | 30.67 | 30.71 |
| Min : Max | 19.7 : 64.7 | 20.3 : 59.7 | 19.7 : 64.7 |
| | | | |
| Baseline BMI categories (kg/m²) [n (%)] | | | |
| Number | 223 | 223 | 446 |
| <30 | 103 (46.2%) | 103 (46.2%) | 206 (46.2%) |
| ≥30 | 120 (53.8%) | 120 (53.8%) | 240 (53.8%) |

| | | | |
|---|---|---|---|
| BMI = Body Mass Index. TZDs = Thiazolidinediones | | | |

Disease characteristics including diabetic history were summarized in Table 4, 5 and 6. The median duration of diabetes was slightly higher for the lixisenatide group (8.12 years) than that for the placebo group (7.28 years). Diabetic chronic complications including diabetic neuropathy, retinopathy and nephropathy were generally compatible with small variations in the proportion of patients in each treatment group. Of note, eleven patients (8 for lixisenatide and 3 for placebo) took GLP-1 receptor agonists prior to the study.

The average daily dose of insulin glargine at baseline (V12,week-1) (see Section 5.3) was 43.44U for the lixisenatide group and 44.24 U for the placebo group. The average dose remained nearly unchanged at randomization (V13) (44.08 U for lixisenatide and 44.95 U for placebo) in both treatment groups.

The duration of usage and the average daily dose of metformin were very similar between the two treatment groups; at baseline, the average dose was 2048.7 mg for the study population. Out of the 72 patients who used TZDs at screening visit, 54 patients continued the TZDs at baseline with an identical usage proportion in both treatment groups (12.1%, Table 7). The discrepancy in the number of patients between the "randomization strata of TZD use" (Table 3) and the "actual TZD use at baseline" was due to randomization strata errors (Table 7). Three patients in lixisenatide group did not used TZDs at randomization, but were randomized with stratification 'TZD = Yes'. Eight patients (6 in lixisenatide and 2 in placebo) used TZDs at randomization, but were randomized with stratification 'TZD = No'.

**Table 4 Disease characteristics at screening or baseline - Safety population**

| | **Placebo (N=223)** | **Lixisenatide (N=223)** | **All (N=446)** |
|---|---|---|---|
| Duration of diabetes (years) | | | |
| Number | 223 | 223 | 446 |
| Mean (SD) | 8.72 (5.82) | 9.62 (6.03) | 9.17 (5.94) |
| Median | 7.28 | 8.12 | 7.93 |
| Min: Max | 1.0 : 30.0 | 1.0 : 31.5 | 1.0: 31.5 |
| | | | |
| Age at onset of type 2 diabetes (years) | | | |
| Number | 223 | 223 | 446 |
| Mean (SD) | 47.3 (10.4) | 46.8 (9.4) | 47.1 (9.9) |
| Median | 49.0 | 46.0 | 47.0 |
| Min: Max | 19 : 75 | 23 : 75 | 19 : 75 |
| | | | |
| History of gestational diabetes [n (%)] | | | |
| Number (Female) | 110 | 114 | 224 |
| Yes (Female) | 10 (9.1%) | 12 (10.5%) | 22 (9.8%) |
| No (Female) | 100 (90.9%) | 102 (89.5%) | 202 (90.2%) |
| | | | |
| Prior use of GLP-1 receptor agonist [n (%)] | | | |
| Number | 223 | 223 | 446 |
| Yes | 3 (1.3%) | 8 (3.6%) | 11 (2.5%) |
| No | 220 (98.7%) | 215 (96.4%) | 435 (97.5%) |
| Diabetic retinopathy [n (%)] | | | |
| Number | 216 | 215 | 431 |
| Yes | 22 (10.2%) | 21 (9.8%) | 43 (10.0%) |
| No | 183 (84.7%) | 174 (80.9%) | 357 (82.8%) |
| Unknown | 11 (5.1%) | 20 (9.3%) | 31(7.2%) |
| | | | |
| Diabetic sensory or motor neuropathy [n (%)] | | | |
| Number | 216 | 215 | 431 |
| Yes | 43 (19.9%) | 29 (13.5%) | 72 (16.7%) |
| No | 165 (76.4%) | 169 (78.6%) | 334 (77.5%) |
| Unknown | 8 (3.7%) | 17 (7.9%) | 25 (5.8%) |
| | | | |
| Diabetic autonomic neuropathy [n (%)] | | | |
| Number | 216 | 215 | 431 |
| Yes | 3 (1.4%) | 3 (1.4%) | 6 (1.4%) |
| No | 202 (93.5%) | 195 (90.7%) | 397 (92.1%) |
| Unknown | 11 (5.1%) | 17 (7.9%) | 28 (6.5%) |
| | | | |
| Diabetic nephropathy [n (%)] | | | |
| Number | 216 | 215 | 431 |
| Yes | 18 (8.3%) | 11 (5.1%) | 29 (6.7%) |
| Microalbuminuria | 13 (6.0%) | 9 (4.2%) | 22 (5.1%) |
| Overt proteinuria | 4 (1.9%) | 1 (0.5%) | 5 (1.2%) |
| Impaired renal function | 1 (0.5%) | 1 (0.5%) | 2 (0.5%) |
| Dialysis or transplantation | 0 | 0 | 0 |
| No | 184 (85.2%) | 183 (85.1%) | 367 (85.2%) |
| Unknown | 14 (6.5%) | 21 (9.8%) | 35 (8.1%) |
| | | | |
| Categorized albumin/creatinine ratio at baseline [n (%)] | | | |
| Number | 223 | 223 | 446 |
| <30 µg/mg creatinine (normal) | 169 (75.8%) | 173 (77.6%) | 342 (76.7%) |
| ≥30 - <300 µg/mg creatinine (microalbuminuria) | 39 (17.5%) | 41 (18.4%) | 80 (17.9%) |
| ≥300 µg/mg creatinine (macroalbuminuria) | 15 (6.7%) | 9 (4.0%) | 24 (5.4%) |
| | | | |
| Creatinine clearance at baseline (ml/min) | | | |
| Number | 223 | 223 | 446 |
| Mean (SD) | 117.90 (48.45) | 116.89 (43.09) | 117.40 (45.80) |
| Median | 112.83 | 109.47 | 110.56 |
| Min : Max | 21.9 : 567.1 | 35.2 : 270.5 | 21.9 : 567.1 |
| Creatinine clearance categories at baseline [n (%)] | | | |
| Number | 223 | 223 | 446 |
| <30 ml/min (severe renal impairment) | 1 (0.4%) | 0 | 1 (0.2%) |
| ≥30 - <50 ml/min (moderate renal impairment) | 1 (0.4%) | 4 (1.8%) | 5 (1.1%) |
| ≥50 - ≤580 ml/min (mild renal impairment) | 32 (14.3%) | 44 (19.7%) | 76 (17.0%) |
| >80 ml/min (no renal impairment) | 189 (84.8%) | 175 (78.5%) | 364 (81.6%) |

| | | | |
|---|---|---|---|
| GLP-1 = Glucagon like peptide-1. Creatinine clearance value is derived using the equation of Cockcroft and Gault. | | | |

**Table 5 Disease characteristics - Average daily insulin glargine dose (U) at baseline and at randomization - Safety population**

| | **Placebo (N=223)** | **Lixisenatide (N=223)** | **All (N=446)** |
|---|---|---|---|
| Average daily dose at baseline ^{a} (Visit 12: Week -1) | | | |
| Number | 223 | 223 | 446 |
| Mean (SD) | 44.24 (19.86) | 43.44 (18.84) | 43.84 (19.34) |
| Median | 42.00 | 42.00 | 42.00 |
| Min : Max | 4.0 : 127.7 | 10.0 : 167.7 | 4.0 : 167.7 |
| | | | |
| Average daily dose at randomization (Visit 13: Week 0) | | | |
| Number | 223 | 223 | 446 |
| Mean (SD) | 44.95 (20.62) | 44.08 (19.63) | 44.51 (20.11) |
| Median | 42.00 | 42.00 | 42.00 |
| Min : Max | 4.0: 130.0 | 10.0 : 176.0 | 4.0 : 176.0 |

| | | | |
|---|---|---|---|
| ^{a} Insulin glargine average daily dose at baseline is the average daily dose for the week prior to Visit 12 which takes place at Week-1. | | | |

**Table 6 Disease characteristics - Metformin at baseline - Safety population**

| | **Placebo (N=223)** | **Lixisenatide (N=223)** | **All (N=446)** |
|---|---|---|---|
| Duration of metformin treatment (years) | | | |
| Number | 223 | 223 | 446 |
| Mean (SD) | 5.23 (4.52) | 5.87 (4.99) | 5.55 (4.76) |
| Median | 3.91 | 4.94 | 4.40 |
| Min : Max | 0.2 : 29.4 | 0.3 : 29.8 | 0.2 : 29.8 |
| Daily dose of metformin at baseline (mg) | | | |
| Number | 223 | 223 | 446 |
| Mean (SD) | 2058.1 (430.6) | 2039.2 (405.3) | 2048.7 (417.8) |
| Median | 2000.0 | 2000.0 | 2000.0 |
| Min : Max | 1500 : 3400 | 1500 : 3400 | 1500 : 3400 |
| Categorized daily dose of metformin at baseline (mg) [n (%)] | | | |
| Number | 223 | 223 | 446 |
| <1500 | 0 | 0 | 0 |
| ≥1500 - < 2500 | 163 (73.1%) | 165 (74.0%) | 328 (73.5%) |
| ≥2500 - < 3000 | 43 (19.3%) | 49 (22.0%) | 92 (20.6%) |
| ≥3000 | 17 (7.6%) | 9 (4.0%) | 26 (5.8%) |

**Table 7 Disease characteristics - Thiazolidinediones (TZDs) at screening and at baseline-Safety population**

| | **Placebo (N=223)** | **Lixisenatide (N=223)** | **All (N=446)** |
|---|---|---|---|
| TZDs use at screening [n (%)] | | | |
| Number | 223 | 223 | 446 |
| Yes | 32 (14.3%) | 40 (17.9%) | 72 (16.1%) |
| No | 191 (85.7%) | 183 (82.1%) | 374 (83.9%) |
| TZDs use at baseline [n (%)] | | | |
| Number | 223 | 223 | 446 |
| Yes | 27 (12.1%) | 27 (12.1%) | 54 (12.1%) |
| No | 196 (87.9%) | 196 (87.9%) | 392 (87.9%) |
| Daily dose of TZDs at baseline (mg) | | | |
| Rosiglitazone | | | |
| Number | 10 | 10 | 20 |
| Mean (SD) | 5.6 (2.1) | 5.6 (2.1) | 5.6 (2.0) |
| Median | 4.0 | 4.0 | 4.0 |
| Min : Max | 4 : 8 | 4 : 8 | 4 : 8 |
| Pioglitazone | | | |
| Number | 17 | 17 | 34 |
| Mean (SD) | 30.9 (11.2) | 28.2 (11.7) | 29.6 (11.4) |
| Median | 30.0 | 30.0 | 30.0 |
| Min : Max | 15 : 45 | 15 : 45 | 15 : 45 |
| Categorized daily dose of TZDs at baseline (mg) [n(%)]^{a} | | | |
| Rosiglitazone | | | |
| Number | 10 | 10 | 20 |
| Low dose | 0 | 0 | 0 |
| Medium dose | 6 (60.0%) | 6 (60.0%) | 12 (60.0%) |
| High dose | 4 (40.0%) | 4 (40.0%) | 8 (40.0%) |
| Pioglitazone | | | |
| Number | 17 | 17 | 34 |
| Low dose | 4 (23.5%) | 6 (35.3%) | 10 (29.4%) |
| Medium dose | 8 (47.1%) | 7 (41.2%) | 15 (44.1%) |
| High dose | 5 (29.4%) | 4 (23.5%) | 9 (26.5%) |

| | | | |
|---|---|---|---|
| TZDs = Thiazolidinediones ^{a}Low dose: 1-2mg/day rosiglitazone or 15 mg/day pioglitazone, Medium dose: 4 mg/day rosiglitazone or 30 mg/day pioglitazone, High dose: 8 mg/day rosiglitazone or 45 mg/day pioglitazone | | | |

Baseline efficacy variables were generally comparable between the two treatment groups for the safety population (Table 8). The study population in the two groups was well matched with regard to the baseline glycemic parameters, including HbA1c, FPG, PPG and 7-point SMPG, with only small differences in the mean values.

**Table 8 Baseline efficacy variables - Safety population**

| | **Placebo (N=223)** | **Lixisenatide (N=223)** | **All (N=446)** |
|---|---|---|---|
| HbA1c (%) | | | |
| Number | 223 | 223 | 446 |
| Mean (SD) | 7.60 (0.54) | 7.56 (0.55) | 7.58 (0.54) |
| Median | 7.40 | 7.50 | 7.50 |
| Min : Max | 6.7 : 9.1 | 6.0: 9.1 | 6.0 : 9.1 |
| | | | |
| Weight (kg) | | | |
| Number | 223 | 223 | 446 |
| Mean (SD) | 86.75 (20.41) | 87.31 (21.76) | 87.03 (21.07) |
| Median | 85.20 | 84.00 | 84.65 |
| Min : Max | 45.6 : 187.3 | 47.5 : 169.4 | 45.6 : 187.3 |
| | | | |
| FPG (mmol/L) | | | |
| Number | 223 | 223 | 446 |
| Mean (SD) | 6.70 (1.97) | 6.55 (1.72) | 6.62 (1.85) |
| Median | 6.33 | 6.33 | 6.33 |
| Min : Max | 3.4 : 16.8 | 3.2 : 12.7 | 3.2 : 16.8 |
| | | | |
| 2-hour postprandial plasma glucose (mmol/L) | | | |
| Number | 221 | 219 | 440 |
| Mean (SD) | 12.79 (3.69) | 12.90 (3.94) | 12.85 (3.81) |
| Median | 13.04 | 12.65 | 12.82 |
| Min : Max | 3.3 : 30.5 | 3.6 : 29.0 | 3.3 : 30.5 |
| Glucose excursion (mmol/L) | | | |
| Number | 221 | 219 | 440 |
| Mean (SD) | 6.33 (3.54) | 6.24 (4.35) | 6.29 (3.96) |
| Median | 6.77 | 6.08 | 6.34 |
| Min : Max | -5.2 : 20.5 | -8.8 : 21.7 | -8.8 : 21.7 |
| Average daily insulin glargine dose (U) | | | |
| Number | 223 | 223 | 446 |
| Mean (SD) | 44.24 (19.86) | 43.44 (18.84) | 43.84 (19.34) |
| Median | 42.00 | 42.00 | 42.00 |
| Min : Max | 4.0: 127.7 | 10.0 : 167.7 | 4.0 : 167.7 |
| Sum of DTSQs score ^{a} | | | |
| Number | 222 | 221 | 443 |
| Mean (SD) | 31.5 (5.1) | 31.7 (4.5) | 31.6 (4.8) |
| Median | 33.0 | 33.0 | 33.0 |
| Min : Max | 8 : 36 | 15 : 36 | 8 : 36 |
| Average of 7-point SMPG (mmol/L) | | | |
| Number | 221 | 221 | 442 |
| Mean (SD) | 8.26 (1.52) | 8.20 (1.47) | 8.23 (1.49) |
| Median | 8.27 | 8.13 | 8.18 |
| Min : Max | 4.4 : 13.3 | 5.3 : 12.9 | 4.4 : 13.3 |

| | | | |
|---|---|---|---|
| FPG = Fasting plasma glucose. SMPG =Self-Monitored Plasma Glucose. DTSQs = Diabetes Treatment Satisfaction Questionnaire (status). Glucose excursion = 2-hour postprandial plasma glucose - plasma glucose 30 minutes prior to the meal test before study drug administration. ^{a} Sum of items 1, 4, 5, 6, 7 and 8 from DTSQs. Note: The baseline for secondary efficacy variables was the last available value prior to the first injection of the double blind IP (lixisenatide or placebo) except for insulin glargine dose (average daily dose at baseline is the average daily dose for the week prior to Visit 12 which takes place at Week -1). | | | |

### 6.1.4 Dosage and duration

The average IP (lixisenatide or placebo) treatment exposure was 155.8 days (22.3 weeks) for the lixisenatide group and 163.4 days (23.3 weeks) for the placebo group (Table 9). Of the 446 patients, 143 (64.1%) patients in the lixisenatide group and 151 (67.7%) patients in the placebo group received at least 169 days (24 weeks) of treatment.

For the lixisenatide group, 196 (87.9%) patients were at the target total daily dose of 20 µg at the end of the 24-week double-blind treatment period (Table 10). For the placebo group, 215 (96.4%) patients were at the target total daily dose of 20 µg at the end of 24-week double-blind treatment period (Table 10).

**Table 9 Exposure - Safety population**

| | **Placebo (N=223)** | **Lixisenatide (N=223)** |
|---|---|---|
| Cumulative duration of treatment exposure (patient years) | 99.8 | 95.1 |
| | | |
| Duration of study treatment (days) | | |
| Number | 223 | 223 |
| Mean (SD) | 163.4 (28.9) | 155.8 (41.2) |
| Median | 169.0 | 169.0 |
| Min : Max | 9 : 211 | 6 : 197 |
| | | |
| Duration of study treatment by category [n (%)] | | |
| Missing | 0 | 0 |
| 1-14 days | 1 (0.4%) | 4 (1.8%) |
| 15-28 days | 4 (1.8%) | 6 (2.7%) |
| 29-56 days | 3 (1.3%) | 8 (3.6%) |
| 57-84 days | 2 (0.9%) | 4 (1.8%) |
| 85-168 days | 62 (27.8%) | 58 (26.0%) |
| >168 days | 151 (67.7%) | 143 (64.1%) |
| | | |
| Cumulative duration of study treatment by category [n (%)] | | |
| Missing | 0 | 0 |
| ≥ 1 day | 223 (100%) | 223 (100%) |
| ≥ 15 days | 222 (99.6%) | 219 (98.2%) |
| ≥ 29 days | 218 (97.8%) | 213 (95.5%) |
| ≥ 57 days | 215 (96.4%) | 205 (91.9%) |
| ≥ 85 days | 213 (95.5%) | 201 (90.1%) |
| ≥ 169 days | 151 (67.7%) | 143 (64.1%) |

| | | |
|---|---|---|
| Duration of exposure = (date of the last double-blind investigational product injection - date of the first double-blind investigational product injection) + 1. | | |

**Table 10 Number (%) of patients by final total daily dose at the end of the double-blind treatment - Safety population**

| **Final dose** | **Placebo (N=223)** | **Lixisenatide (N=223)** |
|---|---|---|
| 10 µg | 2 (0.9%) | 17 (7.6%) |
| 15 µg | 6 (2.7%) | 10 (4.5%) |
| 20 µg | 215 (96.4%) | 196 (87.9%) |

| | | |
|---|---|---|
| Dose = Dose of active drug or volume-matched placebo. Note: Percentages are calculated using the number of safety patients as the denominator. | | |

### 6.2 EFFICACY

### 6.2.1 Primary efficacy endpoint

### Main analysis

Table 11 summarizes the results of the primary efficacy parameter, change from baseline to Week 24 (LOCF) in HbA_{1c} using an ANCOVA analysis.

Insulin glargine treatment during the 12-week run-in phase had resulted in a remarkable reduction in the mean HbA1c value from 8.6% in each group (Table 34) to 7.56% in the lixisenatide group and 7.60% in the placebo group. The mean HbA1c value was further reduced in both treatment groups during the 24-week randomized treatment phase to 6.96% in the lixisenatide group and 7.30% in the placebo group. The least squared (LS) mean change from randomization baseline to Week 24 in HbA1c was -0.71% for the lixisenatide group and -0.40% for the placebo group. The pre-specified primary analysis showed that treatment with lixisenatide resulted in a statistically significant decrease in HbA_{1c} from baseline to Week 24, compared to treatment with placebo (LS mean difference versus the placebo group = -0.32%; p-value <0.0001). Of note, per protocol, insulin dose adjustment to maintain fasting plasma glucose at target was allowed in both treatment groups throughout the study.

**Table 11 Mean change in HbA1c (%) from baseline to Week 24 - mITT population**

| **HbA1c (%)** | **Placebo (N=223)** | **Lixisenatide (N=223)** |
|---|---|---|
| Baseline | | |
| Number | 221 | 215 |
| Mean (SD) | 7.60 (0.54) | 7.56 (0.54) |
| Median | 7.40 | 7.50 |
| Min : Max | 6.7 : 9.1 | 6.0 : 9.1 |
| | | |
| Week 24 (LOCF) | | |
| Number | 221 | 215 |
| Mean (SD) | 7.30 (0.85) | 6.96 (0.81) |
| Median | 7.10 | 6.80 |
| Min : Max | 5.4: 11.2 | 5.4 : 10.4 |
| | | |
| Change from baseline to Week 24 (LOCF) | | |
| Number | 221 | 215 |
| Mean (SD) | -0.30 (0.80) | -0.60 (0.77) |
| Median | -0.40 | -0.70 |
| Min : Max | -3.2 : 2.8 | -2.9 : 2.4 |
| | | |
| LS Mean (SE) ^{a} | -0.40 (0.092) | -0.71 (0.091) |
| LS Mean difference (SE) vs. Placebo ^{a} | - | -0.32 (0.074) |
| 95% CI | - | (-0.463 to -0.171) |
| p-value | | <.0001 |

| | | |
|---|---|---|
| LOCF = Last observation carried forward. TZDs = Thiazolidinediones. ^{a} Analysis of covariance (ANCOVA) model with treatment groups (lixisenatide and placebo), randomization strata of Visit 12 (Week -1) HbA1c (<8.0, ≥8.0%), randomization strata of TZDs use (Yes or No), and country as fixed effects and baseline HbA1c value as a covariate. Note: The analysis excluded measurements obtained after the introduction of rescue medication and/or after the treatment cessation plus 14 days. Patients with both baseline and Week 24 (LOCF) measurements are included. | | |

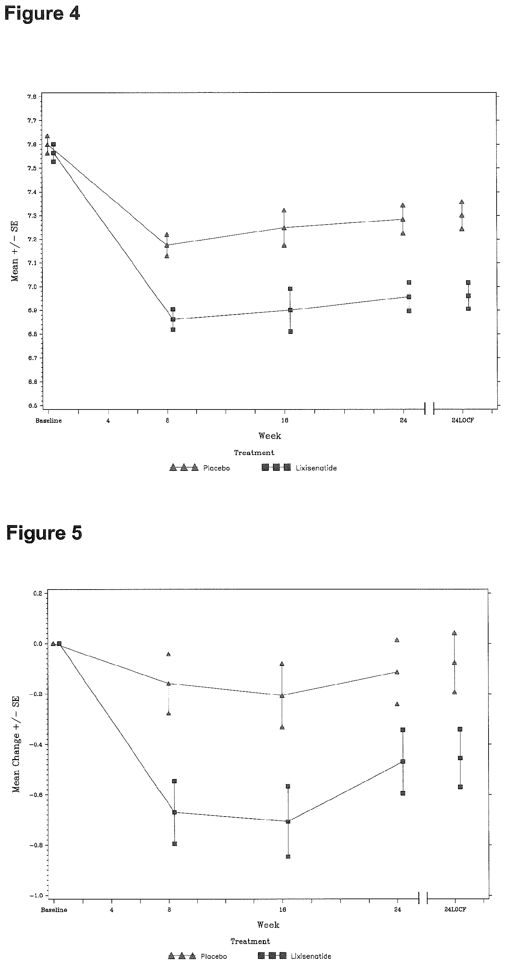

Figures 3 and 4 illustrate the mean (±SE) change from baseline in HbA₁c and the mean (±SE) HbA₁c values by visit during the 24-week double-blind treatment period.

As shown by Figure 3, ,both treatments reached a glycemic plateau from Week 8 through Week 16 and a slight increase in HbA1c was observed during the late phase of the treatment period toward the end.

Table 12 summarizes the proportion of patients with treatment response in HbA_{1c} ≤6.5% or <7% at Week 24, respectively. The analysis of HbA_{1c} responders using the CMH method showed a significant treatment difference between the lixisenatide and placebo groups (p-value < 0.0001 and p-value = 0.0001, respectively) in both HbA1c categories. At Week 24, 32.1% of lixisenatide-treated patients and 16.3% of placebo-treated patients achieved HbA_{1c} values ≤6.5%; 56.3% of patients in the lixisenatide group and 38.5% of patients in the placebo group achieved HbA_{1c} values <7%.

**Table 12 Number (%) of patients with HbA1c value ≤6.5% or <7% respectively at Week 24 - mITT population**

| **HbA1c (%)** | **Placebo (N=223)** | **Lixisenatide (N=223)** |
|---|---|---|
| Number | 221 | 215 |
| ≤6.5% | 36 (16.3%) | 69 (32.1%) |
| >6.5% | 185 (83.7%) | 146 (67.9%) |
| p-value vs. placebo^{a} | - | <0.0001 |
| | | |
| Number | 221 | 215 |
| <7.0% | 85 (38.5%) | 121 (56.3%) |
| ≥7.0% | 136 (61.5%) | 94 (43.7%) |
| p-value vs. placebo^{a} | - | 0.0001 |

| | | |
|---|---|---|
| TZDs = Thiazolidinediones. ^{a} Cochran-Mantel-Haenszel (CMH) method stratified by randomization strata of Visit 12 (Week -1) HbA1c (<8.0, ≥8.0%) and randomization strata of TZDs use (Yes or No). Note: The analysis excluded measurements obtained after the introduction of rescue medication and/or after the treatment cessation plus 14 days. Patients with both baseline and Week 24 (LOCF) measurements are included. | | |

### 6.2.2 Secondary efficacy endpoints

Table 13 -16, and Table 18,19 and 21 summarize the ANCOVA analyses of 2-hour postprandial plasma glucose, glucose excursion, average 7-point SMPG, body weight, insulin glargine dose, FPG and DTSQs scores, respectively. Figure 5, 7-9 illustrate the Mean (±SE) change from baseline in average 7-point SMPG, body weight, insulin glargine dose and FPG over time during the 24 week double-blind treatment period.

The results of the 2-hour postprandial plasma glucose after a standard test-meal showed a statistically significant improvement from baseline to Week 24 in the lixisenatide group compared with the placebo group (LS mean difference versus placebo = -3.16 mmol/L; p-value <.0001, Table 13). Moreover, treatment with lixisenatide substantially decreased post-prandial plasma glucose excursion from Baseline to Week 24 compared to treatment with placebo (LS mean difference = -3.09 mmol/L, 95% CI = -3.842 to -2.331) (Table 14).

**Table 13 Mean change in 2-hour postprandial plasma glucose (mmol/L) from baseline to Week 24 - mITT population**

| **2-hour postprandial plasma glucose (mmol/L)** | **Placebo (N=223)** | **Lixisenatide (N=223)** |
|---|---|---|
| Baseline | | |
| Number | 204 | 194 |
| Mean (SD) | 12.85 (3.75) | 13.02 (3.83) |
| Median | 13.10 | 12.71 |
| Min : Max | 3.3 : 30.5 | 3.6 : 26.2 |
| | | |
| Week 24 (LOCF) | | |
| Number | 204 | 194 |
| Mean (SD) | 13.04 (3.94) | 9.87 (4.24) |
| Median | 13.10 | 9.52 |
| Min : Max | 4.9 : 24.5 | 2.6 : 25.3 |
| | | |
| Change from baseline to Week 24 (LOCF) | | |
| Number | 204 | 194 |
| Mean (SD) | 0.18 (4.48) | -3.15 (5.05) |
| Median | 0.11 | -2.72 |
| Min : Max | -13.0: 18.0 | -16.8 : 10.5 |
| LS Mean (SE) ^{a} | 0.08 (0.481) | -3.09 (0.482) |
| | | |
| LS Mean difference (SE) vs. Placebo ^{a} | - | -3.16 (0.401) |
| 95% CI | - | (-3.951 to -2.375) |
| p-value | | <.0001 |

| | | |
|---|---|---|
| LOCF = Last observation carried forward. TZDs = Thiazolidinediones. ^{a} Analysis of covariance (ANCOVA) model with treatment groups (lixisenatide and placebo), randomization strata of Visit 12 (Week -1) HbA1c (<8.0, ≥8.0%), randomization strata of TZDs use (Yes or No), and country as fixed effects and baseline 2-hour postprandial plasma glucose value as a covariate. Note: The analysis excluded measurements obtained after the introduction of rescue medication and/or after the treatment cessation. Patients with both baseline and Week 24 (LOCF) measurements are included. | | |

**Table 14 Mean change in glucose excursion (mmol/L) from baseline to Week 24 - mITT population**

| **Glucose excursion (mmol/L)** | **Placebo (N=223)** | **Lixisenatide (N=223)** |
|---|---|---|
| Baseline | | |
| Number | 204 | 194 |
| Mean (SD) | 6.37 (3.61) | 6.40 (4.21) |
| Median | 6.74 | 6.28 |
| Min : Max | -5.2: 20.5 | -5.7: 21.7 |
| | | |
| Week 24 (LOCF) | | |
| Number | 204 | 194 |
| Mean (SD) | 6.22 (3.66) | 3.15(4.10) |
| Median | 6.28 | 2.70 |
| Min : Max | -2.7: 20.2 | -4.0: 19.7 |
| | | |
| Change from baseline to Week 24 (LOCF) | | |
| Number | 204 | 194 |
| Mean (SD) | -0.15 (4.33) | -3.26 (5.07) |
| Median | -0.25 | -3.12 |
| Min : Max | -11.4:20.2 | -16.7: 12.7 |
| LS Mean (SE) ^{a} | -0.33 (0.461) | -3.42 (0.462) |
| | | |
| LS Mean difference (SE) vs. Placebo ^{a} | - | -3.09 (0.384) |
| 95% CI | - | (-3.842 to -2.331) |

| | | |
|---|---|---|
| LOCF = Last observation carried forward. TZDs = Thiazolidinediones. Glucose excursion = 2-hour postprandial plasma glucose - plasma glucose 30 minutes prior to the meal test before study drug administration. ^{a} Analysis of covariance (ANCOVA) model with treatment groups (lixisenatide and placebo), randomization strata of Visit 12 (Week -1) HbA1c (<8.0, ≥8.0%), randomization strata of TZDs use (Yes or No), and country as fixed effects and baseline glucose excursion value as a covariate. Note: The analysis excluded measurements obtained after the introduction of rescue medication and/or after the treatment cessation. Patients with both baseline and Week 24 (LOCF) measurements are included. | | |

For the average 7-point SMPG, a statistically significant glucose reduction from baseline to Week 24 was observed in lixisenatide group compared with the placebo group (LS mean difference versus placebo = -0.39 mmol/L; p-value =0.0071) (Table 15). Overall glycemia measured by 7-point SMPG with both treatments was in agreement with the trend of HbA1c over the course of the 24-week treatment period (Figure 4).

**Table 15 Mean change in average 7-point Self Monitored Plasma Glucose (SMPG) (mmol/L) from baseline to Week 24 - mITT population**

| **Average 7-point Self Monitored Plasma Glucose (SMPG) (mmol/L)** | **Placebo (N=223)** | **Lixisenatide (N=223)** |
|---|---|---|
| Baseline | | |
| Number | 214 | 210 |
| Mean (SD) | 8.29 (1.52) | 8.20 (1.45) |
| Median | 8.31 | 8.14 |
| Min : Max | 4.4 : 13.3 | 5.3 : 12.9 |
| | | |
| Week 24 (LOCF) | | |
| Number | 214 | 210 |
| Mean (SD) | 8.21 (1.72) | 7.75 (1.51) |
| Median | 7.95 | 7.53 |
| Min : Max | 4.6 : 14.9 | 4.7 : 14.0 |
| | | |
| Change from baseline to Week 24 (LOCF) | | |
| Number | 214 | 210 |
| Mean (SD) | -0.08 (1.72) | -0.46 (1.66) |
| Median | -0.04 | -0.50 |
| Min : Max | -4.8 : 4.3 | -5.0 : 3.6 |
| LS Mean (SE) ^{a} | -0.08 (0.179) | -0.47 (0.178) |
| | | |
| LS Mean difference (SE) vs. Placebo ^{a} | - | -0.39 (0.146) |
| 95% CI | - | (-0.680 to -0.107) |
| p-value | | 0.0071 |

| | | |
|---|---|---|
| LOCF = Last observation carried forward. TZDs = Thiazolidinediones. ^{a} Analysis of covariance (ANCOVA) model with treatment groups (lixisenatide and placebo), randomization strata of Visit 12 (Week -1) HbAlc (<8.0, ≥8.0%), randomization strata of TZDs use (Yes or No), and country as fixed effects and baseline average 7-point SMPG value as a covariate. Note: The analysis excluded measurements obtained after the introduction of rescue medication and/or after the treatment cessation. Patients with both baseline and Week 24 (LOCF) measurements are included. | | |

As shown on Fig 6 which illustrates the 7-point SMPG by each time point at baseline and endpoint, a profound reduction in post breakfast and a modest decrease in post lunch from baseline to Week 24 were observed in the lixisenatide group compared to that in the placebo group; whereas, it appeared that the decrease in post prandial glucose waned over post dinner and bedtime.

The LS mean body weight change from baseline to Week 24 was 0.28 kg for the lixisenatide-treated patients and 1.16 kg for the placebo-treated patients. A statistically significant less weight gain in the lixisenatide group than in the placebo group was observed (LS mean difference versus placebo = -0.89 kg, p-value = 0.0012) (Table 16). Slightly more lixisenatide-treated patients (5.1%) than placebo-treated patients (3.2%) had a weight loss of 5% or more from baseline to Week 24 (Table 17).

**Table 16 Mean change in body weight (kg) from baseline to Week 24 - mITT population**

| **Body weight (kg)** | **Placebo (N=223)** | **Lixisenatide (N=223)** |
|---|---|---|
| Baseline | | |
| Number | 220 | 217 |
| Mean (SD) | 86.74 (20.54) | 87.47 (21.98) |
| Median | 85.10 | 84.40 |
| Min : Max | 45.6: 187.3 | 47.5 : 169.4 |
| | | |
| Week 24 (LOCF) | | |
| Number | 220 | 217 |
| Mean (SD) | 87.54 (20.74) | 87.45 (22.25) |
| Median | 86.75 | 84.00 |
| Min : Max | 45.7: 183.2 | 49.0 : 173.0 |
| | | |
| Change from baseline to Week 24 (LOCF) | | |
| Number | 220 | 217 |
| Mean (SD) | 0.80 (2.85) | -0.02 (2.76) |
| Median | 0.60 | 0.00 |
| Min : Max | -9.5 : 12.8 | -8.0: 7.9 |
| LS Mean (SE) ^{a} | 1.16 (0.330) | 0.28 (0.331) |
| | | |
| LS Mean difference (SE) vs. Placebo ^{a} | - | -0.89 (0.272) |
| 95% CI | - | (-1.423 to -0.353) |
| p-value | | 0.0012 |

| | | |
|---|---|---|
| LOCF = Last observation carried forward. TZDs = Thiazolidinediones. ^{a} Analysis of covariance (ANCOVA) model with treatment groups (lixisenatide and placebo), randomization strata of Visit 12 (Week -1) HbAlc (<8.0, ≥8.0%), randomization strata of TZDs use (Yes or No), and country as fixed effects and baseline body weight as a covariate. Note: The analysis excluded measurements obtained after the introduction of rescue medication and/or after the treatment cessation plus 3 days. Patients with both baseline and Week 24 (LOCF) measurements are included. | | |

**Table 17 Number (%) of patients with >=5 % weight loss from baseline to Week 24 - mITT population**

| **Weight loss** | **Placebo (N=223)** | | **Lixisenatide (N=223)** | |
|---|---|---|---|---|
| Number | 220 | | 217 | |
| ≥5% | 7 | (3.2%) | 11 | (5.1%) |
| <5%^{a} | 213 | (96.8%) | 206 | (94.9%) |

| | | | | |
|---|---|---|---|---|
| ^{a} Patients with less than 5% weight loss are included in this category, including patients who gained weight. Note: The analysis excluded measurements obtained after the introduction of rescue medication and/or after the treatment cessation plus 3 days. Patients with both baseline and Week 24 (LOCF) measurements are included. | | | | |

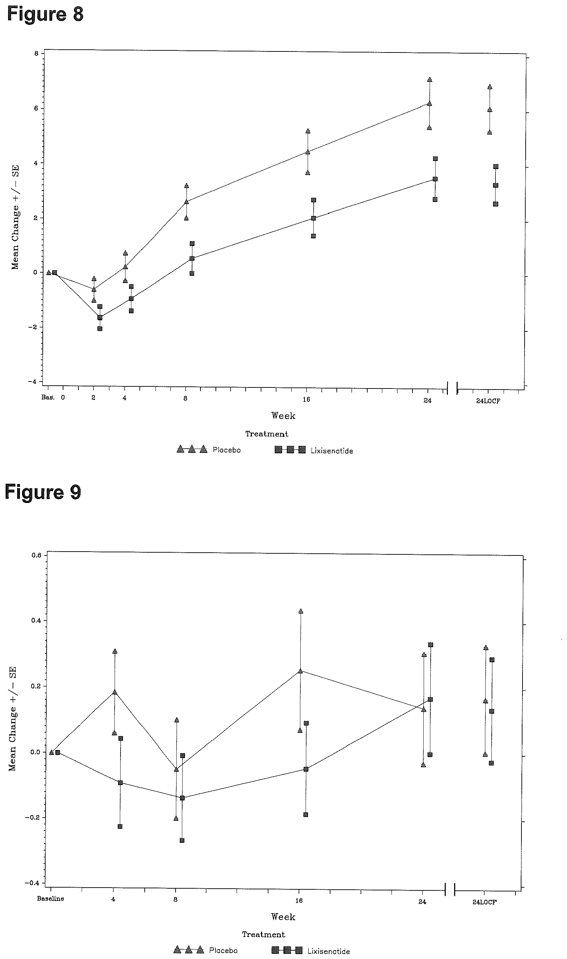

Over the 24 week on-treatment period, the daily insulin dose in both groups increased gradually, which was permitted by the protocol to maintain FPGs between 100 and 80 mg/d (5.6 and 4.4 mmmol/L). However, patients in the lixisenatide group showed a considerably less increase in daily insulin glargine dose (Figure 8) while achieving a greater reduction in HbA1c. The mean change in insulin dose for lixisenatide group at the endpoint (Week 24) reached a statistically significant difference compared with the placebo group (LS mean difference versus placebo = -2.24 U; p-value =0.0300) (Table 18).

**Table 18 Mean change in basal insulin dose (U) from baseline to Week 24 - mITT population**

| **Average daily insulin glargine dose (U)** | **Placebo (N=223)** | **Lixisenatide (N=223)** |
|---|---|---|
| Baseline | | |
| Number | 223 | 222 |
| Mean (SD) | 44.24 (19.86) | 43.41 (18.87) |
| Median | 42.00 | 42.00 |
| Min : Max | 4.0 : 127.7 | 10.0 : 167.7 |
| | | |
| Week 24 (LOCF) | | |
| Number | 223 | 222 |
| Mean (SD) | 50.35 (26.39) | 46.74 (23.83) |
| Median | 46.00 | 44.00 |
| Min : Max | 4.0 : 182.0 | 8.0 : 192.0 |
| Change from baseline to Week 24 (LOCF) | | |
| Number | 223 | 222 |
| Mean (SD) | 6.11 (12.36) | 3.33 (10.22) |
| Median | 4.00 | 2.00 |
| Min : Max | -22.0 : 76.6 | -28.9 : 44.0 |
| LS Mean (SE) ^{a} | 5.34 (1.256) | 3.10 (1.260) |
| | | |
| LS Mean difference (SE) vs. Placebo ^{a} | - | -2.24 (1.029) |
| 95% CI | - | (-4.264 to -0.218) |
| p-value | | 0.0300 |

| | | |
|---|---|---|
| LOCF = Last observation carried forward. TZDs = Thiazolidinediones. ^{a} Analysis of covariance (ANCOVA) model with treatment groups (lixisenatide and placebo), randomization strata of Visit 12 (Week -1) HbA1c (<8.0, ≥8.0%), randomization strata of TZDs use (Yes or No), and country as fixed effects and baseline average daily insulin glargine as a covariate. Note: The analysis excluded measurements obtained after the introduction of rescue medication and/or after the treatment cessation. Patients with both baseline and Week 24 (LOCF) measurements are included. | | |

Patients in either treatment group showed a slight increase in FPG from baseline to Week 24 (LS mean 0.34 mmol/L for lixisenatide versus 0.46 mmol/L for placebo) with no statistically significant difference observed between the lixisenatide and placebo groups (LS mean difference versus placebo = -0.12 mmol/L; p-value =0.5142) (Table 19).

**Table 19 Mean change in fasting plasma glucose (mmol/L) from baseline to Week 24 - mITT population**

| **Fasting plasma glucose (mmol/L)** | **Placebo (N=223)** | **Lixisenatide (N=223)** |
|---|---|---|
| Baseline | | |
| Number | 220 | 214 |
| Mean (SD) | 6.69 (1.98) | 6.56 (1.74) |
| Median | 6.30 | 6.33 |
| Min : Max | 3.4 : 16.8 | 3.2: 12.7 |
| Week 24 (LOCF) | | |
| Number | 220 | 214 |
| Mean (SD) | 6.86 (1.88) | 6.70 (1.79) |
| Median | 6.44 | 6.38 |
| Min : Max | 3.3 : 13.4 | 3.4 : 16.9 |
| Change from baseline to Week 24 (LOCF) | | |
| Number | 220 | 214 |
| Mean (SD) | 0.17 (2.41) | 0.14 (2.30) |
| Median | 0.24 | 0.11 |
| Min : Max | -12.2 : 6.5 | -7.8 : 7.1 |
| LS Mean (SE) ^{a} | 0.46 (0.214) | 0.34 (0.213) |
| LS Mean difference (SE) vs. Placebo ^{a} | - | -0.12 (0.177) |
| 95% CI | - | (-0.463 to 0.232) |
| p-value | | 0.5142 |

| | | |
|---|---|---|
| LOCF = Last observation carried forward. TZDs = Thiazolidinediones. ^{a} Analysis of covariance (ANCOVA) model with treatment groups (lixisenatide and placebo), randomization strata of Visit 12 (Week -1) HbAlc (<8.0, ≥8.0%), randomization strata of TZDs use (Yes or No), and country as fixed effects and baseline fasting plasma glucose value as a covariate. Note: The analysis excluded measurements obtained after the introduction of rescue medication and/or after the treatment cessation plus 1 day. Patients with both baseline and Week 24 (LOCF) measurements are included. | | |

As per the testing strategy adjusting for multiplicity, inferential testing for the percentages of patients requiring rescue therapy at Week 24 were exploratory since the preceding test (FPG) failed to show statistically significant group difference. A total of 2 patients (1 [0.4%] each in the placebo group and lixisenatide group) received a rescue therapy (Table 20).

**Table 20 Number (%) of patients requiring rescue therapy during the 24-week treatment period - mITT population**

| **Requiring rescue therapy** | **Placebo (N=223)** | **Lixisenatide (N=223)** |
|---|---|---|
| Number | 223 | 223 |
| Yes | 1 (0.4%) | 1 (0.4%) |
| No | 222 (99.6%) | 222 (99.6%) |
| **Requiring rescue therapy** | **Placebo (N=223)** | **Lixisenatide (N=223)** |
| p-value vs. placebo^{a} | - | 1.0000 |

| | | |
|---|---|---|
| TZDs = Thiazolidinediones ^{a} Cochran-Mantel-Haenszel (CMH) method stratified by randomization strata of Visit 12 (Week -1) HbAlc (<8.0 or ≥8.0%) and TZDs use (Yes, No). | | |

**Table 21 Mean change in the Diabetes Treatment Satisfaction Questionnaire (DTSQs) score from baseline to Week 24 - mITT population**

| **Diabetes Treatment Satisfaction Questionnaire (DTSQs) score** | **Placebo (N=223)** | **Lixisenatide (N=223)** |
|---|---|---|
| Baseline | | |
| Number | 209 | 201 |
| Mean (SD) | 31.58 (5.07) | 31.70 (4.47) |
| Median | 33.00 | 33.00 |
| Min : Max | 8.0 : 36.0 | 15.0 : 36.0 |
| | | |
| Week 24 (LOCF) | | |
| Number | 209 | 201 |
| Mean (SD) | 32.02 (5.15) | 32.42 (4.84) |
| Median | 34.00 | 34.00 |
| Min : Max | 9.0 : 36.0 | 5.0 : 36.0 |
| | | |
| Change from baseline to Week 24 (LOCF) | | |
| Number | 209 | 201 |
| Mean (SD) | 0.45 (5.41) | 0.71 (4.56) |
| Median | 0.00 | 0.00 |
| Min : Max | -25.0 : 19.0 | -26.0 : 16.0 |
| LS Mean (SE) ^{a} | 0.65 (0.545) | 0.88 (0.543) |
| | | |
| LS Mean difference (SE) vs. Placebo ^{a} | - | 0.23 (0.451) |
| 95% CI | - | (-0.660 to 1.114) |

| | | |
|---|---|---|
| LOCF = Last observation carried forward. TZDs = Thiazolidinediones. DTSQs = Diabetes Treatment Satisfaction Questionnaire (status). ^{a} Analysis of covariance (ANCOVA) model with treatment groups (lixisenatide and placebo), randomization strata of Visit 12 (Week -1) HbA1c (<8.0, ≥8.0%), randomization strata of TZDs use (Yes or No), and country as fixed effects and baseline treatment satisfaction score as a covariate. Note: The analysis excluded measurements obtained after the introduction of rescue medication and/or after the treatment cessation + 3 days. DTSQs score: Sum of items 1, 4, 5, 6, 7 and 8 from DTSQs. Patients with both baseline and Week 24 (LOCF) measurements are included. | | |

### 6.3 SAFETY

An overview of the adverse events observed during the on-treatment period is provided in Table 22. The proportion of the patients with treatment emergent adverse events (TEAEs) was 79.8% for lixisenatide group and 68.2% for placebo group. The disproportionate number of patients with TEAEs in the lixisenatide group was primarily driven by the GI related AEs (39.9% for lixisenatide versus 16.1% for placebo). Two patients (both on placebo) had TEAEs leading to death. The percentage of patients who experienced serious TEAEs was higher in the lixisenatide group (7.6%) than in the placebo group (4.5%), without a notable increased occurrence in any specific System Organ Classes (SOC). The percentage of patients with TEAEs leading to treatment discontinuation was 8.5% in the lixisenatide group compared with 3.6% in the placebo group. The most common TEAEs leading to treatment discontinuation were nausea and vomiting in the lixisenatide group (9 patients [4.0 %]), while no patient in the placebo group discontinued the treatment due to nausea or vomiting. Tables 23, 24, and 25 summarize TEAEs leading to death, serious TEAEs, and TEAEs leading to treatment discontinuation by primary SOC, High Level Group Term (HLGT), High Level Term (HLT) and Preferred Term (PT).

Table 35 in the appendix presents the incidences of TEAEs occurring at least 1% of patients in any treatment group during the on-treatment period. For both treatment groups, hypoglycaemia was the most frequently reported TEAE (61 [27.4%] for lixisenatide and 43 [19.3%] for placebo). Aside from hypoglycemia, the most common TEAE in the lixisenatide group was nausea (61 patients [27.4%] for lixisenatide versus 11 patients [4.9%] for placebo), followed by headache (22 patients [9.9%] for lixisenatide versus 8 [3.6%] for placebo) and vomiting (21 patients [9.4%] for lixisenatide versus 3 [1.3%] for placebo).

**Table 22 Overview of adverse event profile: treatment emergent adverse events during the on-treatment period - Safety population**

| | **Placebo (N=223)** | **Lixisenatide (N=223)** |
|---|---|---|
| Patients with any TEAE | 152 (68.2%) | 178 (79.8%) |
| Patients with any serious TEAE | 10 (4.5%) | 17 (7.6%) |
| Patients with any TEAE leading to death | 2 (0.9%) | 0 |
| Patients with any TEAE leading to permanent treatment discontinuation | 8 (3.6%) | 19 (8.5%) |

| | | |
|---|---|---|
| TEAE: Treatment Emergent Adverse Event n (%) = number and percentage of patients with at least one adverse event Note: On-treatment period = the time from the first dose of double-blind study medication up to 3 days after the last dose administration. | | |

**Table 23 Number (%) of patients experiencing TEAE(s) leading to death by primary SOC, HLGT, HLT, and PT during the on-treatment period - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=223)** | **(N=223)** |
| Any class | 2 (0.9%) | 0 |
| | | |
| NEOPLASMS BENIGN, MALIGNANT AND UNSPECIFIED (INCL CYSTS AND POLYPS) | 1 (0.4%) | 0 |
| HLGT: Plasma cell neoplasms | 1 (0.4%) | 0 |
| HLT: Multiple myelomas | 1 (0.4%) | 0 |
| Multiple myeloma | 1 (0.4%) | 0 |
| CARDIAC DISORDERS | 1 (0.4%) | 0 |
| HLGT: Coronary artery disorders | 1 (0.4%) | 0 |
| HLT: Ischaemic coronary artery disorders | 1 (0.4%) | 0 |
| Myocardial infarction | 1 (0.4%) | 0 |

| | | |
|---|---|---|
| TEAE: Treatment Emergent Adverse Event, SOC: System Organ Class, HLGT: High Level Group Term, HLT: High Level Term, PT: Preferred Term. MedDRA version: 14.0. n (%) = number and percentage of patients with at least one TEAE leading to death. Note: On-treatment period = the time from the first dose of double-blind study medication up to 3 days after the last dose administration. Table sorted by SOC internationally agreed order and HLGT, HLT, PT alphabetic order. | | |

**Table 24 Number (%) of patients experiencing serious TEAE(s) presented by primary SOC, HLGT, HLT, and PT during the on-treatment period - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=223)** | **(N=223)** |
| Any class | 10 (4.5%) | 17 (7.6%) |
| | | |
| INFECTIONS AND INFESTATIONS | 1 (0.4%) | 3 (1.3%) |
| HLGT: Infections - pathogen unspecified | 1 (0.4%) | 3 (1.3%) |
| HLT: Abdominal and gastrointestinal infections | 0 | 1 (0.4%) |
| Gastroenteritis | 0 | 1 (0.4%) |
| HLT: Lower respiratory tract and lung infections | 1 (0.4%) | 1 (0.4%) |
| Pneumonia | 1 (0.4%) | 1 (0.4%) |
| HLT: Sepsis, bacteraemia, viraemia and fungaemia NEC | 0 | 1 (0.4%) |
| Urosepsis | 0 | 1 (0.4%) |
| | | |
| NEOPLASMS BENIGN, MALIGNANT AND UNSPECIFIED (INCL CYSTS AND POLYPS) | 1 (0.4%) | 1 (0.4%) |
| HLGT: Gastrointestinal neoplasms malignant and unspecified | 0 | 1 (0.4%) |
| HLT: Colonic neoplasms malignant | 0 | 1 (0.4%) |
| Colon cancer | 0 | 1 (0.4%) |
| HLGT: Plasma cell neoplasms | 1 (0.4%) | 0 |
| HLT: Multiple myelomas | 1 (0.4%) | 0 |
| Multiple myeloma | 1 (0.4%) | 0 |
| | | |
| METABOLISM AND NUTRITION DISORDERS | 0 | 2 (0.9%) |
| HLGT: Electrolyte and fluid balance conditions | 0 | 1 (0.4%) |
| HLT: Total fluid volume decreased | 0 | 1 (0.4%) |
| Dehydration | 0 | 1 (0.4%) |
| HLGT: Glucose metabolism disorders (incl diabetes mellitus) | 0 | 1 (0.4%) |
| HLT: Hypoglycaemic conditions NEC | 0 | 1 (0.4%) |
| Hypoglycaemic unconsciousness | 0 | 1 (0.4%) |
| | | |
| PSYCHIATRIC DISORDERS | 1 (0.4%) | 1 (0.4%) |
| HLGT: Schizophrenia and other psychotic disorders | 0 | 1 (0.4%) |
| HLT: Schizophrenia NEC | 0 | 1 (0.4%) |
| Schizophrenia, paranoid type | 0 | 1 (0.4%) |
| HLGT: Suicidal and self-injurious behaviours NEC | 1 (0.4%) | 0 |
| HLT: Suicidal and self-injurious behaviour | 1 (0.4%) | 0 |
| Suicide attempt | 1 (0.4%) | 0 |
| | | |
| NERVOUS SYSTEM DISORDERS | 0 | 2 (0.9%) |
| HLGT: Central nervous system vascular disorders | 0 | 2 (0.9%) |
| HLT: Central nervous system haemorrhages and cerebrovascular accidents | 0 | 1 (0.4%) |
| Cerebrovascular accident | 0 | 1 (0.4%) |
| HLT: Transient cerebrovascular events | 0 | 1 (0.4%) |
| Transient ischaemic attack | 0 | 1 (0.4%) |
| | | |
| CARDIAC DISORDERS | 4 (1.8%) | 4 (1.8%) |
| HLGT: Coronary artery disorders | 4 (1.8%) | 3 (1.3%) |
| HLT: Ischaemic coronary artery disorders | 4 (1.8%) | 3 (1.3%) |
| Acute myocardial infarction | 1 (0.4%) | 0 |
| Angina pectoris | 0 | 1 (0.4%) |
| Angina unstable | 2 (0.9%) | 2 (0.9%) |
| Myocardial infarction | 1 (0.4%) | 0 |
| HLGT: Heart failures | 0 | 1 (0.4%) |
| HLT: Heart failures NEC | 0 | 1 (0.4%) |
| Cardiac failure congestive | 0 | 1 (0.4%) |
| | | |
| VASCULAR DISORDERS | 1 (0.4%) | 2 (0.9%) |
| HLGT: Decreased and nonspecific blood pressure disorders and shock | 0 | 1 (0.4%) |
| HLT: Circulatory collapse and shock | 0 | 1 (0.4%) |
| Hypovolaemic shock | 0 | 1 (0.4%) |
| HLGT: Embolism and thrombosis | 1 (0.4%) | 0 |
| HLT: Peripheral embolism and thrombosis | 1 (0.4%) | 0 |
| Deep vein thrombosis | 1 (0.4%) | 0 |
| HLGT: Vascular hypertensive disorders | 0 | 1 (0.4%) |
| HLT: Vascular hypertensive disorders NEC | 0 | 1 (0.4%) |
| Hypertension | 0 | 1 (0.4%) |
| RESPIRATORY, THORACIC AND MEDIASTINAL DISORDERS | 1 (0.4%) | 1 (0.4%) |
| HLGT: Bronchial disorders (excl neoplasms) | 0 | 1 (0.4%) |
| HLT: Bronchospasm and obstruction | 0 | 1 (0.4%) |
| Asthma | 0 | 1 (0.4%) |
| HLGT: Lower respiratory tract disorders (excl obstruction and infection) | 1 (0.4%) | 0 |
| HLT: Pulmonary oedemas | 1 (0.4%) | 0 |
| Pulmonary oedema | 1 (0.4%) | 0 |
| | | |
| GASTROINTESTINAL DISORDERS | 2 (0.9%) | 2 (0.9%) |
| HLGT: Abdominal hernias and other abdominal wall conditions | 0 | 1 (0.4%) |
| HLT: Abdominal hernias, site unspecified | 0 | 1 (0.4%) |
| Abdominal hernia | 0 | 1 (0.4%) |
| HLGT: Gastrointestinal haemorrhages NEC | 1 (0.4%) | 0 |
| HLT: Non-site specific gastrointestinal haemorrhages | 1 (0.4%) | 0 |
| Upper gastrointestinal haemorrhage | 1 (0.4%) | 0 |
| HLGT: Gastrointestinal inflammatory conditions | 1 (0.4%) | 0 |
| HLT: Colitis (excl infective) | 1 (0.4%) | 0 |
| Colitis ischaemic | 1 (0.4%) | 0 |
| HLGT: Gastrointestinal vascular conditions | 0 | 1 (0.4%) |
| HLT: Haemorrhoids and gastrointestinal varices (excl oesophageal) | 0 | 1 (0.4%) |
| Haemorrhoids | 0 | 1 (0.4%) |
| | | |
| HEPATOBILIARY DISORDERS | 1 (0.4%) | 0 |
| HLGT: Gallbladder disorders | 1 (0.4%) | 0 |
| HLT: Cholecystitis and cholelithiasis | 1 (0.4%) | 0 |
| Cholecystitis acute | 1 (0.4%) | 0 |
| | | |
| SKIN AND SUBCUTANEOUS TISSUE DISORDERS | 1 (0.4%) | 0 |
| HLGT: Angioedema and urticaria | 1 (0.4%) | 0 |
| HLT: Angioedemas | 1 (0.4%) | 0 |
| Angioedema | 1 (0.4%) | 0 |
| | | |
| MUSCULOSKELETAL AND CONNECTIVE TISSUE DISORDERS | 1 (0.4%) | 0 |
| HLGT: Joint disorders | 1 (0.4%) | 0 |
| HLT: Osteoarthropathies | 1 (0.4%) | 0 |
| Osteoarthritis | 1 (0.4%) | 0 |
| INJURY, POISONING AND PROCEDURAL COMPLICATIONS | 0 | 2 (0.9%) |
| HLGT: Injuries NEC | 0 | 2 (0.9%) |
| HLT: Cerebral injuries NEC | 0 | 1 (0.4%) |
| Subdural haematoma | 0 | 1 (0.4%) |
| HLT: Site specific injuries NEC | 0 | 1 (0.4%) |
| Head injury | 0 | 1 (0.4%) |
| | | |
| SURGICAL AND MEDICAL PROCEDURES | 0 | 2 (0.9%) |
| HLGT: Vascular therapeutic procedures | 0 | 2 (0.9%) |
| HLT: Arterial therapeutic procedures (excl aortic) | 0 | 2 (0.9%) |
| Coronary angioplasty | 0 | 1 (0.4%) |
| Coronary arterial stent insertion | 0 | 1 (0.4%) |

| | | |
|---|---|---|
| TEAE: Treatment Emergent Adverse Event, SOC: System Organ Class, HLGT: High Level Group Term, HLT: High Level Term, PT: Preferred Term. MedDRA version: 14.0. n (%) = number and percentage of patients with at least one serious TEAE. Note: On-treatment period = the time from the first dose of double-blind study medication up to 3 days after the last dose administration. Table sorted by SOC internationally agreed order and HLGT, HLT, PT alphabetic order. | | |

**Table 25 Number (%) of patients experiencing TEAE(s) leading to permanent treatment discontinuation by primary SOC, HLGT, HLT, and PT during the on-treatment period - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=223)** | **(N=223)** |
| Any class | 8 (3.6%) | 19 (8.5%) |
| | | |
| NEOPLASMS BENIGN, MALIGNANT AND UNSPECIFIED (INCL CYSTS AND POLYPS) | 1 (0.4%) | 1 (0.4%) |
| HLGT: Breast neoplasms malignant and unspecified (incl nipple) | 0 | 1 (0.4%) |
| HLT: Breast and nipple neoplasms malignant | 0 | 1 (0.4%) |
| Breast cancer metastatic | 0 | 1 (0.4%) |
| HLGT: Plasma cell neoplasms | 1 (0.4%) | 0 |
| HLT: Multiple myelomas | 1 (0.4%) | 0 |
| Multiple myeloma | 1 (0.4%) | 0 |
| | | |
| METABOLISM AND NUTRITION DISORDERS | 0 | 1 (0.4%) |
| HLGT: Glucose metabolism disorders (incl diabetes mellitus) | 0 | 1 (0.4%) |
| HLT: Hypoglycaemic conditions NEC | 0 | 1 (0.4%) |
| Hypoglycaemic unconsciousness | 0 | 1 (0.4%) |
| NERVOUS SYSTEM DISORDERS | 0 | 1 (0.4%) |
| HLGT: Movement disorders (incl parkinsonism) | 0 | 1 (0.4%) |
| HLT: Tremor (excl congenital) | 0 | 1 (0.4%) |
| Tremor | 0 | 1 (0.4%) |
| HLGT: Neurological disorders NEC | 0 | 1 (0.4%) |
| HLT: Neurological signs and symptoms NEC | 0 | 1 (0.4%) |
| Dizziness | 0 | 1 (0.4%) |
| | | |
| CARDIAC DISORDERS | 3 (1.3%) | 1 (0.4%) |
| HLGT: Coronary artery disorders | 3 (1.3%) | 0 |
| HLT: Ischaemic coronary artery disorders | 3 (1.3%) | 0 |
| Acute myocardial infarction | 1 (0.4%) | 0 |
| Angina unstable | 1 (0.4%) | 0 |
| Myocardial infarction | 1 (0.4%) | 0 |
| HLGT: Heart failures | 0 | 1 (0.4%) |
| HLT: Heart failures NEC | 0 | 1 (0.4%) |
| Cardiac failure congestive | 0 | 1 (0.4%) |
| | | |
| VASCULAR DISORDERS | 0 | 1 (0.4%) |
| HLGT: Vascular disorders NEC | 0 | 1 (0.4%) |
| HLT: Peripheral vascular disorders NEC | 0 | 1 (0.4%) |
| Flushing | 0 | 1 (0.4%) |
| | | |
| RESPIRATORY, THORACIC AND MEDIASTINAL DISORDERS | 1 (0.4%) | 1 (0.4%) |
| HLGT: Bronchial disorders (excl neoplasms) | 0 | 1 (0.4%) |
| HLT: Bronchospasm and obstruction | 0 | 1 (0.4%) |
| Asthma | 0 | 1 (0.4%) |
| HLGT: Lower respiratory tract disorders (excl obstruction and infection) | 1 (0.4%) | 0 |
| HLT: Pulmonary oedemas | 1 (0.4%) | 0 |
| Pulmonary oedema | 1 (0.4%) | 0 |
| | | |
| GASTROINTESTINAL DISORDERS | 0 | 10 (4.5%) |
| HLGT: Abdominal hernias and other abdominal wall conditions | 0 | 1 (0.4%) |
| HLT: Abdominal hernias, site unspecified | 0 | 1 (0.4%) |
| Abdominal hernia | 0 | 1 (0.4%) |
| HLGT: Gastrointestinal motility and defaecation conditions | 0 | 1 (0.4%) |
| HLT: Diarrhoea (excl infective) | 0 | 1 (0.4%) |
| Diarrhoea | 0 | 1 (0.4%) |
| HLGT: Gastrointestinal signs and symptoms | 0 | 9 (4.0%) |
| HLT: Flatulence, bloating and distension | 0 | 1 (0.4%) |
| Abdominal distension | 0 | 1 (0.4%) |
| HLT: Gastrointestinal and abdominal pains (excl oral and throat) | 0 | 1 (0.4%) |
| Abdominal pain upper | 0 | 1 (0.4%) |
| HLT: Nausea and vomiting symptoms | 0 | 9 (4.0%) |
| Nausea | 0 | 6 (2.7%) |
| Vomiting | 0 | 5 (2.2%) |
| | | |
| HEPATOBILIARY DISORDERS | 1 (0.4%) | 0 |
| HLGT: Gallbladder disorders | 1 (0.4%) | 0 |
| HLT: Cholecystitis and cholelithiasis | 1 (0.4%) | 0 |
| Cholecystitis acute | 1 (0.4%) | 0 |
| | | |
| SKIN AND SUBCUTANEOUS TISSUE DISORDERS | 0 | 1 (0.4%) |
| HLGT: Angioedema and urticaria | 0 | 1 (0.4%) |
| HLT: Urticarias | 0 | 1 (0.4%) |
| Urticaria | 0 | 1 (0.4%) |
| | | |
| MUSCULOSKELETAL AND CONNECTIVE TISSUE DISORDERS | 1 (0.4%) | 1 (0.4%) |
| HLGT: Joint disorders | 0 | 1 (0.4%) |
| HLT: Joint related signs and symptoms | 0 | 1 (0.4%) |
| Arthralgia | 0 | 1 (0.4%) |
| HLGT: Musculoskeletal and connective tissue disorders NEC | 1 (0.4%) | 0 |
| HLT: Musculoskeletal and connective tissue pain and discomfort | 1 (0.4%) | 0 |
| Back pain | 1 (0.4%) | 0 |
| | | |
| RENAL AND URINARY DISORDERS | 1 (0.4%) | 0 |
| HLGT: Renal disorders (excl nephropathies) | 1 (0.4%) | 0 |
| HLT: Renal failure and impairment | 1 (0.4%) | 0 |
| Renal failure | 1 (0.4%) | 0 |
| | | |
| GENERAL DISORDERS AND ADMINISTRATION SITE CONDITIONS | 0 | 3 (1.3%) |
| HLGT: Administration site reactions | 0 | 2 (0.9%) |
| HLT: Injection site reactions | 0 | 2 (0.9%) |
| Injection site reaction | 0 | 1 (0.4%) |
| Injection site swelling | 0 | 1 (0.4%) |
| HLGT: General system disorders NEC | 0 | 1 (0.4%) |
| HLT: Asthenic conditions | 0 | 1 (0.4%) |
| Asthenia | 0 | 1 (0.4%) |
| INVESTIGATIONS | 1 (0.4%) | 0 |
| HLGT: Gastrointestinal investigations | 1 (0.4%) | 0 |
| HLT: Digestive enzymes | 1 (0.4%) | 0 |
| Lipase increased | 1 (0.4%) | 0 |

| | | |
|---|---|---|
| TEAE: Treatment Emergent Adverse Event, SOC: System Organ Class, HLGT: High Level Group Term, HLT: High Level Term, PT: Preferred Term. MedDRA version: 14.0. n (%) = number and percentage of patients with at least one TEAE leading to permanent treatment discontinuation. Note: On-treatment period = the time from the first dose of double-blind study medication up to 3 days after the last dose administration. Table sorted by SOC internationally agreed order and HLGT, HLT, PT alphabetic order. | | |

Hypoglycemia was further analyzed according to the protocol definition (see Section 3.2.2). During the on-treatment period, 50 (22.4%) lixisenatide-treated patients reported 87 symptomatic hypoglycemic events and 30 (13.5%) placebo-treated patients reported 53 symptomatic hypoglycemic events (Table 26). The incidence rate for symptomatic hypoglycemia was 89.8 per 100 patient years for lixisenatide and 52.2 per 100 patient years for placebo. The incidence rate for symptomatic hypoglycemia confirmed by a BG < 60mg/dL was 79.5 per 100 patient years for lixisenatide and 44.3 per 100 patient years for placebo.

In addition, 24 patients (11 for lixisenatide and 13 for placebo), who reported hypoglycemic TEAEs (Table 35), were not included in Table 26 because of not fulfilling the protocol definition; among them, 23 reported hypoglycemia with a blood glucose value above 60 mg/dl (3.3 mmol/L) and one patient did not test blood glucose and spontaneously recovered without any treatment with carbohydrate.

**Table 26 Summary of symptomatic hypoglycemia during the on-treatment period - Safety population**

| **Type** | **Placebo (N=223)** | **Lixisenatide (N=223)** |
|---|---|---|
| Total patient years | 101.6 | 96.9 |
| | | |
| Any symptomatic hypoglycemia | | |
| Number of patients with events, n (%) | 30 (13.5%) | 50 (22.4%) |
| Number of patients with events per 100 patient years^{a} | 29.5 | 51.6 |
| Number of events | 53 | 87 |
| Number of events per 100 patient years^{b} | 52.2 | 89.8 |
| | | |
| Blood glucose <60 mg/dL | | |
| Number of patients with events, n (%) | 26 (11.7%) | 45 (20.2%) |
| Number of patients with events per 100 patient years^{a} | 25.6 | 46.4 |
| Number of events | 45 | 77 |
| Number of events per 100 patient years^{b} | 44.3 | 79.5 |
| No blood glucose reported | | |
| Number of patients with events, n (%) | 6 (2.7%) | 8 (3.6%) |
| Number of patients with events per 100 patient years^{a} | 5.9 | 8.3 |
| Number of events | 8 | 10 |
| Number of events per 100 patient years^{b} | 7.9 | 10.3 |

| | | |
|---|---|---|
| ^{a} Calculated as (number of patients with events*100 divided by total exposure + 3 days in patient years). ^{b} Calculated as (number of events*100 divided by total exposure + 3 days in patient years). Symptomatic hypoglycemia = Symptomatic hypoglycemia as defined per protocol. Note: On- treatment period = the time from the first dose of double-blind study medication up to 3 days after the last dose administration. | | |

During the on-treatment period, one patient [lixisenatide (0.4%)] in the entire safety population, reported 1 severe symptomatic hypoglycemic event per protocol definition (see Section 3.2.2). This 71 year-old female patient had a serious TEAE of hypoglycaemic unconsciousness (Tables 24 & 27). Five days after the first IP administration, around 13:30, while walking, she experienced loss of consciousness associated with sweating and numbness in lips. She received help from people passing by, ate chocolate and then checked her blood sugar which was 134mg/dL at 14:00. Her last meal before the event was the same day at 8:50. The investigator assessed the event as possibly related to the IP and suggested that delayed meal may be an alternative explanation for the hypoglycemia. IP was permanently discontinued due to this event.

**Table 27 Summary of severe symptomatic hypoglycemia during the on-treatment period - Safety population**

| **Type** | **Placebo (N=223)** | **Lixisenatide (N=223)** |
|---|---|---|
| Total patient years | 101.6 | 96.9 |
| | | |
| Any severe symptomatic hypoglycemia | | |
| Number of patients with events, n (%) | 0 | 1 (0.4%) |
| Number of patients with events per 100 patient years^{a} | 0 | 1.0 |
| Number of events | 0 | 1 |
| Number of events per 100 patient years^{b} | 0 | 1.0 |
| | | |
| Blood glucose <36 mg/dL | | |
| Number of patients with events, n (%) | 0 | 0 |
| Number of patients with events per 100 patient years^{a} | 0 | 0 |
| Number of events | 0 | 0 |
| Number of events per 100 patient years^{b} | 0 | 0 |
| | | |
| No blood glucose reported | | |
| Number of patients with events, n (%) | 0 | 1 (0.4%) |
| Number of patients with events per 100 patient years^{a} | 0 | 1.0 |
| Number of events | 0 | 1 |
| Number of events per 100 patient years^{b} | 0 | 1.0 |

| | | |
|---|---|---|
| ^{a} Calculated as (number of patients with events* 100 divided by total exposure + 3 days in patient years). ^{b} Calculated as (number of events*100 divided by total exposure + 3 days in patient years). Severe symptomatic hypoglycemia = Severe symptomatic hypoglycemia as defined per protocol. Note: On- treatment period = the time from the first dose of double-blind study medication up to 3 days after the last dose administration. | | |

Fifteen patients (6.7%) from lixisenatide group and 5 patients (2.2%) from placebo group experienced injection site reaction AEs during the on-treatment period (Table 28). The injection site reaction AEs were identified by searching for the term "injection site" in both the investigator reported AE PTs and PTs coded from the ARAC diagnosis. None of the reactions were serious or severe in intensity. Nonetheless, two patients in the lixisenatide group had an injection site related TEAE leading to IP discontinuation.

**Table 28 Number (%) of patients experiencing injection site reactions during the on-treatment period - Safety population**

| **Event source** | **Placebo (N=223)** | **Lixisenatide (N=223)** |
|---|---|---|
| **Preferred Term** | | |
| Any injection site reactions | 5 (2.2%) | 15 (6.7%) |
| | | |
| Investigator reported PTs | 4 (1.8%) | 14 (6.3%) |
| Injection site haematoma | 2 (0.9%) | 5 (2.2%) |
| Injection site pain | 2 (0.9%) | 2 (0.9%) |
| Injection site erythema | 0 | 1 (0.4%) |
| Injection site inflammation | 0 | 1 (0.4%) |
| Injection site nodule | 0 | 1 (0.4%) |
| Injection site reaction | 0 | 3 (1.3%) |
| Injection site swelling | 0 | 3 (1.3%) |
| | | |
| PTs by ARAC diagnosis | 1 (0.4%) | 4 (1.8%) |
| Injection site reaction | 1 (0.4%) | 4 (1.8%) |

| | | |
|---|---|---|
| ARAC = Allergic Reaction Assessment Committee. PT= Preferred Term. Note: On-treatment period = the time from the first dose of double-blind study medication up to 3 days after the last dose administration. | | |

During the on-treatment period, 25 events from 19 patients were reported as suspected allergic events by investigators and sent to ARAC for adjudication. Of these, 4 events from 4 patients (3 [1.3%] lixisenatide-treated patients and 1 [0.4%] placebo-treated patient) were adjudicated as allergic reactions by the ARAC, and 3 of these events (two events from lixisenatide group and one event from placebo group) were adjudicated as possibly related to the IP (Table 29):
- Patient 840212004 (lixisenatide): A 51-year-old female patient with an ongoing medical history of dyslipidemia, hypothyroidism and allergy to drugs, reported a TEAE of urticaria of moderate intensity on 30-May-2010 (Day 4 on the IP). The patient complained of local reaction of itching and swelling at the injection site (abdomen), which was worsened and observed by the investigator during a site visit on 04-June-2010. After giving IP injection at the office on 04-June-2010, the patient broke out in all over body rash with local swelling and local and general itching. Her BP measured during the reaction was 110/68 mmHg and HR 68 bpm, which were within the range of her vital sign's records. She was promptly fully-recovered after receiving the treatment with betamethasone i.m. and oral diphenhydramine at the site. The IP was discontinued on 04-June-2010. The causal assessment was related per the investigator. The allergic reaction was adjudicated as urticaria and possibly related to the IP by the ARAC.
- Patient 616206009 (lixisenatide): A 49-year-old female patient with a medical history of hypertension, dyslipidemia, and no allergy history reported a TEAE of allergic reaction on 27-Jan-2011 (Day 22 on the IP). Following administration with the IP, the patient presented with a rash on the arms and legs and she complained of generalized itching and flushing. The IP was temporarily stopped for 6 days and the patient recovered without a curative treatment. The IP was reintroduced with the lowest dose and titrated to the target dose of 20 ug. The patient completed the study without an additional allergic reaction reported. The causal assessment was related to the IP per the investigator. The allergic reaction was adjudicated as urticaria and possibly related to the IP by the ARAC.
- Patient 170201023 (placebo): A 69-year-old male patient with a medical history of bilateral keratoconus, gout, vitiligo, and no allergy history reported a TEAE of skin rash on 08-Nov-2010 (Day 4 on the IP). The patient presented with skin rash erythematous lesions on his right ram, left region of the abdomen and left elbow. He also complained of itching and local swelling at the injection site. He was treated with calamine and camphor lotion, and was gradually recovered in 3 weeks on 25-Nov-2010. The patient completed the study and the causal assessment was related to the IP per the investigator. The allergic reaction was adjudicated as dermatitis and possibly related to the IP by the ARAC.

**Table 29 Number (%) of patients with events adjudicated as allergic reaction by ARAC during the on-treatment period - Safety population**

| **Relationship to study treatment (by ARAC)** | **MedDRA coded term (PT) for ARAC diagnosis** | **ARAC Diagnosis** | **Placebo (N=223)** | **Lixisenatide (N=223)** |
|---|---|---|---|---|
| All | Events adjudicated as an allergic reaction by ARAC | | | |
| | | | 1 (0.4%) | 3 (1.3%) |
| | Dermatitis | DERMATITIS | 1 (0.4%) | 0 |
| | Urticaria | URTICARIA (HIVES) | 0 | 3 (1.3%) |
| | Events adjudicated as an allergic reaction by ARAC | | | |
| Possibly Related to IP | | | | |
| | | | 1 (0.4%) | 2 (0.9%) |
| | Dermatitis | DERMATITIS | 1 (0.4%) | 0 |
| | Urticaria | URTICARIA (HIVES) | 0 | 2 (0.9%) |
| Not related to IP | Events adjudicated as an allergic reaction by ARAC | | 0 | 1 (0.4%) |
| | Urticaria | URTICARIA (HIVES) | 0 | 1 (0.4%) |

| | | | | |
|---|---|---|---|---|
| ARAC = Allergic Reaction Assessment Committee. IP = Investigational Product. PT = Preferred Term. Note: On-treatment period = the time from the first dose of double-blind study medication up to 3 days after the last dose administration. | | | | |

Per protocol, any increase in amylase and/or lipase above twice the upper limit of normal range (ULN) that had been confirmed by a repeat measurement was to be monitored and documented on a pre-specified form: "adverse event form for suspected pancreatitis". During the on-treatment period, 5 (2.2%) lixisenatide-treated patients and 10 (4.5%) placebo-treated patient reported 34 TEAEs on the pre-specified AE form (Table 30). Of these, one TEAE of "suspected pancreatitis" of mild intensity was reported in the placebo group. In addition, 4 patients (2 on placebo and 2 on lixisenatide) had an unconfirmed elevation of lipase reported as TEAEs in the regular AE form (Table 35).

Patients who had at least one value of lipase or amylase ≥ 3 ULN during the on-treatment period are summarized in Table 31. Thirteen patients (4 [1.8%] patients in the lixisenatide group and 9 [4.1%] in the placebo group) with elevated lipase (≥ 3ULN) were observed. One patient in the placebo group had elevated amylase (≥ 3ULN), and none did in the lixisenatide group.

**Table 30 Number (%) of patients with TEAE reported on the specific adverse event form for suspected pancreatitis during the on-treatment period - Safety population**

| **Preferred Term** | **Placebo (N=223)** | **Lixisenatide (N=223)** |
|---|---|---|
| Any | 10 (4.5%) | 5 (2.2%) |
| Blood amylase increased | 3 (1.3%) | 1 (0.4%) |
| Lipase increased | 7 (3.1%) | 4 (1.8%) |
| Pancreatic enzymes increased | 1 (0.4%) | 2 (0.9%) |
| Pancreatitis | 1 (0.4%) | 0 |

| | | |
|---|---|---|
| n (%) = number and percentage of patients with any cases reported on the AE form for suspected pancreatitis along with complementary form. Note: On- treatment period = the time from the first dose of double-blind study medication up to 3 days after the last dose administration. | | |

**Table 31 Pancreatic enzymes: Number (%) of patients with at least one post-baseline PCSA during the on-treatment period according to baseline status - Safety population**

| **Laboratory parameter** | **Placebo (N=223)** | **Lixisenatide (N=223)** |
|---|---|---|
| **Baseline** | | |
| **By PCSA criteria n/N1 (%)** | | |
| Lipase (IU/L) | | |
| Total^{*} | | |
| ≥ 3 ULN | 9/221 (4.1%) | 4/219 (1.8%) |
| Normal/Missing | | |
| ≥ 3 ULN | 6/218 (2.8%) | 3/218 (1.4%) |
| | | |
| Amylase (IU/L) | | |
| Total^{*} | | |
| ≥ 3 ULN | 1/221 (0.5%) | 0/219 |
| Normal/Missing | | |
| ≥ 3 ULN | 0/220 | 0/219 |

| | | |
|---|---|---|
| PCSA: Potentially Clinically Significant Abnormalities, ULN= Upper limit of normal. *Regardless of baseline. Note: On-treatment period = the time from the first dose of double-blind study medication up to 3 days after the last dose administration. The number (n) represents the subset of the total number of patients who met the criterion in question at least once. The denominator (/N1) for each parameter within a treatment group is the number of patients for the treatment group who had that parameter assessed post-baseline by baseline PCSA status. Only the worsening of the worst case for each patient is presented by baseline status. | | |

Per protocol, any calcitonin value ≥20 pg/mL confirmed by a repeat measurement was to be monitored and reported on the pre-specified adverse event form for "increased calcitonin ≥20 pg/mL". During the on-treatment period, 2 patients on placebo, and no patient on lixisenatide, reported 2 TEAEs of blood calcitonin increase (Table 32). In addition, 2 TEAEs of calcitonin increase, which were <20 pg/mL, were reported in regular AE form (Table 35) from 2 patients in the placebo group.

**Table 32 Number (%) of patients with TEAE reported on the specific adverse event form for increased calcitonin (≥ 20 ng/L) during the on-treatment period - Safety population**

| **Preferred Term** | **Placebo (N=223)** | **Lixisenatide (N=223)** |
|---|---|---|
| Any | 2 (0.9%) | 0 |
| Blood calcitonin increased | 2 (0.9%) | 0 |

| | | |
|---|---|---|
| n (%) = number and percentage of patients with any cases reported on the AE form for increased calcitonin ≥ 20ng/L. Note: On-treatment period = the time from the first dose of double-blind study medication up to 3 days after the last dose administration. | | |

Patients with at least one serum calcitonin measured during the on-treatment period are summarized in Table 33 according to the 4 categories of calcitonin level at baseline. No patients in the lixisenatide group had calcitonin values ≥20 ng/L over the on-treatment period (Table 33).

**Table 33 Serum calcitonin - Number (%) of patients by pre-defined categories during the on-treatment period according to baseline category - Safety population**

| **Laboratory criteria** | | |
|---|---|---|
| **Baseline status** | **Placebo** | **Lixisenatide** |
| **Post-baseline** | **(N=223)** | **(N=223)** |
| Calcitonin (ng/L) | | |
| Total^{*} | | |
| ≤ULN | 198/215 (92.1%) | 185/206 (89.8%) |
| >ULN - <20 ng/L | 15/215 (7.0%) | 21/206 (10.2%) |
| ≥20 ng/L - <50 ng/L | 2/215 (0.9%) | 0/206 |
| ≥50 ng/L | 0/215 | 0/206 |
| Missing | | |
| ≤ULN | 8/8 (100%) | 4/4 (100%) |
| >ULN - <20 ng/L | 0/8 | 0/4 |
| ≥20 ng/L - <50 ng/L | 0/8 | 0/4 |
| ≥50 ng/L | 0/8 | 0/4 |
| ≤ULN | | |
| ≤ULN | 187/194 (96.4%) | 178/188 (94.7%) |
| >ULN - <20 ng/L | 7/194 (3.6%) | 10/188 (5.3%) |
| ≥20 ng/L - <50 ng/L | 0/194 | 0/188 |
| ≥50 ng/L | 0/194 | 0/188 |
| >ULN - <20 ng/L | | |
| ≤ULN | 3/12 (25.0%) | 3/14 (21.4%) |
| >ULN - <20 ng/L | 8/12 (66.7%) | 11/14 (78.6%) |
| ≥20 ng/L - <50 ng/L | 1/12 (8.3%) | 0/14 |
| ≥50 ng/L | 0/12 | 0/14 |
| ≥20 ng/L - <50 ng/L | | |
| ≤ULN | 0/1 | 0/0 |
| >ULN - <20 ng/L | 0/1 | 0/0 |
| ≥20 ng/L - <50 ng/L | 1/1 (100%) | 0/0 |
| ≥50 ng/L | 0/1 | 0/0 |
| ≥50 ng/L | | |
| ≤ULN | 0/0 | 0/0 |
| >ULN - <20 ng/L | 0/0 | 0/0 |
| ≥20 ng/L - <50 ng/L | 0/0 | 0/0 |
| ≥50 ng/L | 0/0 | 0/0 |

| | | |
|---|---|---|
| ULN= Upper limit of normal *Regardless of baseline. Note: On-treatment period = the time from the first dose of double-blind study medication up to 3 days after the last dose administration. The numerator represents the number of patients who were in the pre-specified categories at post-baseline in each baseline category. The denominator for each parameter within a treatment group is the number of patients for the treatment group who had that parameter assessed post-baseline by baseline status. A patient is counted only in the worst category. | | |

### 7 APPENDIX

**Table 34 Mean chance in HbA1c (%) from baseline by visit - mITT population**

| | **Observed data** | | | | | | | **Change from baseline** | | | | | | |
|---|---|---|---|---|---|---|---|---|---|---|---|---|---|---|
| **Treatment Time point** | **N** | **Mean** | **SD** | **SE** | **Median** | **Min** | **Max** | **N** | **Mean** | **SD** | **SE** | **Median** | **Min** | **Max** |
| Placebo (N=223) | | | | | | | | | | | | | | |
| Screening | 223 | 8.60 | 0.80 | 0.053 | 8.60 | 7.0 | 10.0 | | | | | | | |
| Week-1 | 221 | 7.70 | 0.54 | 0.036 | 7.60 | 7.0 | 9.0 | | | | | | | |
| Baseline | 223 | 7.60 | 0.54 | 0.036 | 7.40 | 6.7 | 9.1 | | | | | | | |
| Week 8 | 210 | 7.17 | 0.65 | 0.045 | 7.10 | 5.7 | 9.3 | 210 | -0.43 | 0.54 | 0.037 | -0.40 | -2.7 | 1.2 |
| Week 16 | 91 | 7.25 | 0.71 | 0.074 | 7.10 | 6.2 | 9.7 | 91 | -0.33 | 0.61 | 0.064 | -0.30 | -1.9 | 1.6 |
| Week 24 | 208 | 7.28 | 0.86 | 0.059 | 7.10 | 5.4 | 11.2 | 208 | -0.32 | 0.81 | 0.056 | -0.40 | -3.2 | 2.8 |
| Week 24 (LOCF) | 221 | 7.30 | 0.85 | 0.057 | 7.10 | 5.4 | 11.2 | 221 | -0.30 | 0.80 | 0.054 | -0.40 | -3.2 | 2.8 |
| Lixisenatide (N=223) | | | | | | | | | | | | | | |
| Screening | 223 | 8.60 | 0.80 | 0.053 | 8.60 | 7.0 | 10.0 | | | | | | | |
| Week-1 | 219 | 7.69 | 0.52 | 0.035 | 7.60 | 7.0 | 9.0 | | | | | | | |
| Baseline | 223 | 7.56 | 0.55 | 0.037 | 7.50 | 6.0 | 9.1 | | | | | | | |
| Week 8 | 205 | 6.86 | 0.61 | 0.043 | 6.70 | 5.4 | 9.0 | 205 | -0.71 | 0.54 | 0.037 | -0.70 | -2.3 | 1.2 |
| Week 16 | 87 | 6.90 | 0.84 | 0.090 | 6.70 | 5.4 | 9.8 | 87 | -0.71 | 0.74 | 0.079 | -0.70 | -2.7 | 1.8 |
| Week 24 | 190 | 6.96 | 0.83 | 0.060 | 6.80 | 5.4 | 10.4 | 190 | -0.62 | 0.80 | 0.058 | -0.70 | -2.9 | 2.4 |
| Week 24 (LOCF) | 215 | 6.96 | 0.81 | 0.055 | 6.80 | 5.4 | 10.4 | 215 | -0.60 | 0.77 | 0.053 | -0.70 | -2.9 | 2.4 |

| | | | | | | | | | | | | | | |
|---|---|---|---|---|---|---|---|---|---|---|---|---|---|---|
| LOCF = Last observation carried forward. Note: The analysis excluded measurements obtained after the introduction of rescue medication and/or after the treatment cessation plus 14 days. | | | | | | | | | | | | | | |

**Table 35 Number (%) of patients experiencing common TEAE(s) (PT ≥1% in any treatment group) presented by primary SOC, HLGT, HLT and PT during the on-treatment period - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=223)** | **(N=223)** |
| Any class | 152 (68.2%) | 178 (79.8%) |
| | | |
| INFECTIONS AND INFESTATIONS | 59 (26.5%) | 63 (28.3%) |
| HLGT: Infections - pathogen unspecified | 43 (19.3%) | 44 (19.7%) |
| HLT: Abdominal and gastrointestinal infections | 7 (3.1%) | 5 (2.2%) |
| Gastroenteritis | 6 (2.7%) | 5 (2.2%) |
| HLT: Lower respiratory tract and lung infections | 5 (2.2%) | 2 (0.9%) |
| Bronchitis | 3 (1.3%) | 1 (0.4%) |
| HLT: Upper respiratory tract infections | 27 (12.1%) | 26 (11.7%) |
| Nasopharyngitis | 12 (5.4%) | 11 (4.9%) |
| Pharyngitis | 3 (1.3%) | 0 |
| Sinusitis | 5 (2.2%) | 4 (1.8%) |
| Upper respiratory tract infection | 4 (1.8%) | 11 (4.9%) |
| HLT: Urinary tract infections | 2 (0.9%) | 10 (4.5%) |
| Urinary tract infection | 2 (0.9%) | 6 (2.7%) |
| HLGT: Viral infectious disorders | 19 (8.5%) | 16 (7.2%) |
| HLT: Herpes viral infections | 4 (1.8%) | 2 (0.9%) |
| Herpes zoster | 3 (1.3%) | 0 |
| HLT: Influenza viral infections | 14 (6.3%) | 11 (4.9%) |
| Influenza | 14 (6.3%) | 11 (4.9%) |
| HLT: Viral infections NEC | 3 (1.3%) | 3 (1.3%) |
| Viral infection | 3 (1.3%) | 2 (0.9%) |
| | | |
| BLOOD AND LYMPHATIC SYSTEM DISORDERS | 2 (0.9%) | 11 (4.9%) |
| HLGT: Anaemias nonhaemolytic and marrow depression | 1 (0.4%) | 6 (2.7%) |
| HLT: Anaemias NEC | 1 (0.4%) | 5 (2.2%) |
| Anaemia | 1 (0.4%) | 5 (2.2%) |
| | | |
| METABOLISM AND NUTRITION DISORDERS | 51 (22.9%) | 70 (31.4%) |
| HLGT: Appetite and general nutritional disorders | 2 (0.9%) | 4 (1.8%) |
| HLT: Appetite disorders | 2 (0.9%) | 4 (1.8%) |
| Decreased appetite | 2 (0.9%) | 4 (1.8%) |
| HLGT: Glucose metabolism disorders (incl diabetes mellitus) | 45 (20.2%) | 61 (27.4%) |
| HLT: Hypoglycaemic conditions NEC | 44 (19.7%) | 61 (27.4%) |
| Hypoglycaemia | 43 (19.3%) | 61 (27.4%) |
| PSYCHIATRIC DISORDERS | 10 (4.5%) | 5 (2.2%) |
| HLGT: Anxiety disorders and symptoms | 5 (2.2%) | 3 (1.3%) |
| HLT: Anxiety symptoms | 5 (2.2%) | 3 (1.3%) |
| Anxiety | 5 (2.2%) | 2 (0.9%) |
| | | |
| NERVOUS SYSTEM DISORDERS | 26 (11.7%) | 46 (20.6%) |
| HLGT: Headaches | 9 (4.0%) | 24 (10.8%) |
| HLT: Headaches NEC | 8 (3.6%) | 23 (10.3%) |
| Headache | 8 (3.6%) | 22 (9.9%) |
| HLGT: Movement disorders (incl parkinsonism) | 4 (1.8%) | 13 (5.8%) |
| HLT: Tremor (excl congenital) | 4 (1.8%) | 13 (5.8%) |
| Tremor | 4 (1.8%) | 13 (5.8%) |
| HLGT: Neurological disorders NEC | 15 (6.7%) | 20 (9.0%) |
| HLT: Neurological signs and symptoms NEC | 7 (3.1%) | 12 (5.4%) |
| Dizziness | 6 (2.7%) | 12 (5.4%) |
| HLT: Sensory abnormalities NEC | 3 (1.3%) | 5 (2.2%) |
| Hypoaesthesia | 2 (0.9%) | 4 (1.8%) |
| | | |
| EYE DISORDERS | 8 (3.6%) | 7 (3.1%) |
| HLGT: Ocular infections, irritations and inflammations | 4 (1.8%) | 3 (1.3%) |
| HLT: Conjunctival infections, irritations and inflammations | 4 (1.8%) | 0 |
| Conjunctivitis | 4 (1.8%) | 0 |
| | | |
| EAR AND LABYRINTH DISORDERS | 3 (1.3%) | 2 (0.9%) |
| HLGT: Inner ear and VIIIth cranial nerve disorders | 3 (1.3%) | 2 (0.9%) |
| HLT: Inner ear signs and symptoms | 3 (1.3%) | 2 (0.9%) |
| Vertigo | 3 (1.3%) | 2 (0.9%) |
| | | |
| VASCULAR DISORDERS | 10 (4.5%) | 11 (4.9%) |
| HLGT: Vascular hypertensive disorders | 8 (3.6%) | 6 (2.7%) |
| HLT: Vascular hypertensive disorders NEC | 7 (3.1%) | 6 (2.7%) |
| Hypertension | 6 (2.7%) | 6 (2.7%) |
| | | |
| RESPIRATORY, THORACIC AND MEDIASTINAL DISORDERS | 11 (4.9%) | 17 (7.6%) |
| HLGT: Respiratory disorders NEC | 8 (3.6%) | 11 (4.9%) |
| HLT: Coughing and associated symptoms | 8 (3.6%) | 6 (2.7%) |
| Cough | 8 (3.6%) | 5 (2.2%) |
| GASTROINTESTINAL DISORDERS | 36 (16.1%) | 89 (39.9%) |
| HLGT: Dental and gingival conditions | 6 (2.7%) | 2 (0.9%) |
| HLT: Dental pain and sensation disorders | 3 (1.3%) | 1 (0.4%) |
| Toothache | 3 (1.3%) | 1 (0.4%) |
| HLGT: Gastrointestinal conditions NEC | 4 (1.8%) | 2 (0.9%) |
| HLT: Gastrointestinal mucosal dystrophies and secretion | | |
| disorders | 3 (1.3%) | 1 (0.4%) |
| Hyperchlorhydria | 3 (1.3%) | 1 (0.4%) |
| HLGT: Gastrointestinal inflammatory conditions | 3 (1.3%) | 5 (2.2%) |
| HLT: Gastritis (excl infective) | 2 (0.9%) | 4 (1.8%) |
| Gastritis | 2 (0.9%) | 4 (1.8%) |
| HLGT: Gastrointestinal motility and defaecation conditions | 10 (4.5%) | 24 (10.8%) |
| HLT: Diarrhoea (excl infective) | 7(3.1%) | 16 (7.2%) |
| Diarrhoea | 7 (3.1%) | 15 (6.7%) |
| HLT: Gastrointestinal atonic and hypomotility disorders NEC | 3 (1.3%) | 8 (3.6%) |
| Constipation | 3 (1.3%) | 6 (2.7%) |
| HLGT: Gastrointestinal signs and symptoms | 17 (7.6%) | 76 (34.1%) |
| HLT: Flatulence, bloating and distension | 3 (1.3%) | 6 (2.7%) |
| Abdominal distension | 2 (0.9%) | 4(1.8%) |
| HLT: Gastrointestinal and abdominal pains (excl oral and throat) | 3 (1.3%) | 14 (6.3%) |
| Abdominal pain | 2 (0.9%) | 7 (3.1%) |
| Abdominal pain upper | 1 (0.4%) | 7 (3.1%) |
| HLT: Nausea and vomiting symptoms | 13 (5.8%) | 67 (30.0%) |
| Nausea | 11 (4.9%) | 61 (27.4%) |
| Vomiting | 3 (1.3%) | 21 (9.4%) |
| | | |
| SKIN AND SUBCUTANEOUS TISSUE DISORDERS | 10 (4.5%) | 16 (7.2%) |
| HLGT: Epidermal and dermal conditions | 8 (3.6%) | 6 (2.7%) |
| HLT: Rashes, eruptions and exanthems NEC | 3 (1.3%) | 1 (0.4%) |
| Rash | 3 (1.3%) | 1 (0.4%) |
| HLGT: Skin appendage conditions | 1 (0.4%) | 9 (4.0%) |
| HLT: Apocrine and eccrine gland disorders | 1 (0.4%) | 8 (3.6%) |
| Hyperhidrosis | 1 (0.4%) | 7 (3.1%) |
| | | |
| MUSCULOSKELETAL AND CONNECTIVE TISSUE DISORDERS | 21 (9.4%) | 28 (12.6%) |
| HLGT: Joint disorders | 9 (4.0%) | 7 (3.1%) |
| HLT: Joint related signs and symptoms | 7 (3.1%) | 5 (2.2%) |
| Arthralgia | 6 (2.7%) | 5 (2.2%) |
| HLGT: Muscle disorders | 3 (1.3%) | 7 (3.1%) |
| HLT: Muscle pains | 1 (0.4%) | 7 (3.1%) |
| Myalgia | 1 (0.4%) | 7 (3.1%) |
| HLGT: Musculoskeletal and connective tissue disorders NEC | 9 (4.0%) | 14 (6.3%) |
| HLT: Musculoskeletal and connective tissue pain and discomfort | 7 (3.1%) | 14 (6.3%) |
| Back pain | 2 (0.9%) | 6 (2.7%) |
| Musculoskeletal pain | 2 (0.9%) | 3 (1.3%) |
| Pain in extremity | 3 (1.3%) | 6 (2.7%) |
| GENERAL DISORDERS AND ADMINISTRATION SITE CONDITIONS | 24 (10.8%) | 34 (15.2%) |
| HLGT: Administration site reactions | 5 (2.2%) | 14 (6.3%) |
| HLT: Injection site reactions | 4 (1.8%) | 14 (6.3%) |
| Injection site haematoma | 2 (0.9%) | 5 (2.2%) |
| Injection site reaction | 0 | 3 (1.3%) |
| Injection site swelling | 0 | 3 (1.3%) |
| HLGT: Body temperature conditions | 4 (1.8%) | 4 (1.8%) |
| HLT: Febrile disorders | 4 (1.8%) | 4 (1.8%) |
| Pyrexia | 4 (1.8%) | 4 (1.8%) |
| HLGT: General system disorders NEC | 15 (6.7%) | 19 (8.5%) |
| HLT: Asthenic conditions | 6 (2.7%) | 14 (6.3%) |
| Asthenia | 3 (1.3%) | 8 (3.6%) |
| Fatigue | 3 (1.3%) | 4 (1.8%) |
| HLT: Oedema NEC | 4 (1.8%) | 2 (0.9%) |
| Oedema peripheral | 4 (1.8%) | 1 (0.4%) |
| | | |
| INVESTIGATIONS | 22 (9.9%) | 18 (8.1%) |
| HLGT: Endocrine investigations (incl sex hormones) | 5 (2.2%) | 0 |
| HLT: Gastrointestinal, pancreatic and APUD hormone analyses | 4 (1.8%) | 0 |
| Blood calcitonin increased | 4 (1.8%) | 0 |
| HLGT: Gastrointestinal investigations | 11 (4.9%) | 7 (3.1%) |
| HLT: Digestive enzymes | 11 (4.9%) | 7 (3.1%) |
| Blood amylase increased | 3 (1.3%) | 1 (0.4%) |
| Lipase increased | 9 (4.0%) | 6 (2.7%) |
| HLGT: Metabolic, nutritional and blood gas investigations | 6 (2.7%) | 3 (1.3%) |
| HLT: Carbohydrate tolerance analyses (incl diabetes) | 5 (2.2%) | 3 (1.3%) |
| Blood glucose decreased | 5 (2.2%) | 3 (1.3%) |
| INJURY, POISONING AND PROCEDURAL COMPLICATIONS | 6 (2.7%) | 11 (4.9%) |
| HLGT: Injuries NEC | 5 (2.2%) | 8 (3.6%) |
| HLT: Non-site specific injuries NEC | 3 (1.3%) | 5 (2.2%) |
| Fall | 1 (0.4%) | 5 (2.2%) |

| | | |
|---|---|---|
| TEAE: Treatment Emergent Adverse Event, SOC: System Organ Class, HLGT: High Level Group Term, HLT: High Level Term, PT: Preferred Term. MedDRA version: 14.0. n (%) = number and percentage of patients with at least one TEAE. Note: On-treatment period = the time from the first dose of double-blind study medication up to 3 days after the last dose administration. Table sorted by SOC internationally agreed order and HLGT, HLT, PT by alphabetic order. Only SOC with at least one PT ≥ 1% in at least one group are presented. | | |

## Claims

1. A pharmaceutical combination for use in the treatment of a diabetes type 2 patient, wherein the diabetes type 2 is insufficiently controlled by at least one oral antidiabetic drug, said combination comprising
(a) desPro³⁶Exendin-4(1-39)-Lys₆-NH₂ or/and a pharmaceutically acceptable salt thereof,
(b) insulin glargine or/and pharmaceutically acceptable salt thereof, and
(c) metformin or/and a pharmaceutically acceptable salt thereof, wherein the treatment of the diabetes type 2 patient comprises the steps:
(i) administration of compounds (b) and (c) for at least 4 weeks, with the proviso that compound (a) is not administered, and
(ii) continuing treatment by administration of compounds (a), (b) and (c),
wherein at the onset of step (i), the patient has a 2 hours postprandial plasma glucose concentration of at least 12 mmol/L, and a glucose excursion of at least 5 mmol/L, wherein the glucose excursion is the difference of the 2 hours postprandial plasma glucose concentration and plasma glucose concentration 30 minutes prior to a meal test, and wherein the amount of compound (b) to be administered in steps (i) or/and (ii) is adjusted so that a predetermined fasting plasma glucose level or/and a predetermined self monitored plasma glucose level is reached or at least approximated.

2. The pharmaceutical combination for use of claim 1, wherein step (i) comprises administration of compounds (b) and (c) for at least 4 weeks, at least 8 weeks, at least 12 weeks, or at least 16 weeks.

3. The pharmaceutical combination for use of any one of the preceding claims, wherein steps (i) or/and (ii) further comprise the administration of a thiazolidinedione.

4. The pharmaceutical combination for use of any one of the preceding claims, wherein the amount of compound (b) to be administered in steps (i) or/and (ii) is adjusted on the basis of daily measurements of plasma glucose concentration.

5. The pharmaceutical combination for use of any one of the preceding claims, wherein the amount of compound (b) to be administered in steps (i) or/and (ii) is adjusted so that a fasting plasma glucose level of about 4.4 mmol/l to about 5.6 mmol/l or/and a self monitored plasma glucose level of about 8 mmol/l is reached or at least approximated.

6. The pharmaceutical combination for use according to any one of the preceding claims, wherein the patient to be treated is obese, and wherein the patient to be treated preferably has a body mass index of at least 30 kg/m².

7. The pharmaceutical combination for use according to any one of the preceding claims, wherein the patient to be treated is an adult patient.

8. The pharmaceutical combination for use according to any one of the preceding claims, wherein the patient to be treated does not receive a treatment by an insulin or/and a pharmaceutically acceptable salt thereof at the onset of step (i).

9. The pharmaceutical combination for use of any one of the preceding claims, wherein in the patient to be treated, diabetes mellitus type 2 has been diagnosed at least 1 year or at least 2 years before onset of therapy.

10. The pharmaceutical combination for use of any one of the preceding claims, wherein the patient to be treated has a HbA_{1c} value of about 7 to about 10%.

11. The pharmaceutical combination for use of any one of the preceding claims, wherein at the onset of step (i), the patient has a fasting plasma glucose concentration of at least 8 mmol/L.

12. The pharmaceutical combination for use of any one of the preceding claims, wherein at the onset of step (i), the patient has a 2 hours postprandial plasma glucose concentration of at least 14 mmol/L.

13. The pharmaceutical combination for use of any one of the preceding claims, wherein (a) the desPro³⁶Exendin-4(1-39)-Lys₆-NH₂ or/and the pharmaceutically acceptable salt thereof is prepared for parenteral administration, (b) the insulin glargine or/and the pharmaceutically acceptable salt thereof is prepared for parenteral administration, or/and (c) the metformin or/and the pharmaceutically acceptable salt thereof is prepared for oral administration.

## Patentansprüche

1. Pharmazeutische Kombination zur Verwendung bei der Behandlung eines Patienten mit Diabetes vom Typ 2, wobei der Diabetes vom Typ 2 durch mindestens ein orales Antidiabetikum unzureichend gesteuert wird, wobei die Kombination Folgendes umfasst:
(a) desPro³⁶Exendin-4(1-39)-Lys₆-NH₂ oder/und ein pharmazeutisch akzeptables Salz davon,
(b) Insulin glargin oder/und ein pharmazeutisch akzeptables Salz davon und
(c) Metformin oder/und ein pharmazeutisch akzeptables Salz davon,
wobei die Behandlung des Patienten mit Diabetes vom Typ 2 die folgenden Schritte umfasst:
(i) die Verabreichung der Verbindungen (b) und (c) über mindestens 4 Wochen, mit der Maßgabe, dass die Verbindung (a) nicht verabreicht wird, und
(ii) die Fortsetzung der Behandlung durch Verabreichung der Verbindungen (a), (b) und (c),
wobei der Patient zu Beginn von Schritt (i) eine 2-Stunden-postprandiale Plasmaglucosekonzentration von mindestens 12 mmol/l und eine Glucoseexkursion von mindestens 5 mmol/l hat, wobei es sich bei der Glucoseexkursion um die Differenz zwischen der 2-Stunden-postprandialen Plasmaglucosekonzentration und der Plasmaglucosekonzentration 30 Minuten vor einer Testmahlzeit handelt und wobei die Menge an in den Schritten (i) oder/und (ii) zu verabreichender Verbindung (b) so eingestellt wird, dass ein vorbestimmter Nüchternplasmaglucosespiegel oder/und ein vorbestimmter selbstüberwachter Plasmaglucosespiegel erreicht oder zumindest nahezu erreicht wird.

2. Pharmazeutische Kombination zur Verwendung nach Anspruch 1, wobei Schritt (i) die Verabreichung der Verbindungen (b) und (c) über mindestens 4 Wochen, mindestens 8 Wochen, mindestens 12 Wochen oder mindestens 16 Wochen umfasst.

3. Pharmazeutische Kombination zur Verwendung nach einem der vorhergehenden Ansprüche, wobei die Schritte (i) oder/und (ii) weiterhin die Verabreichung eines Thiazolidindions umfassen.

4. Pharmazeutische Kombination zur Verwendung nach einem der vorhergehenden Ansprüche, wobei die in den Schritten (i) oder/und (ii) zu verabreichende Menge an Verbindung (b) auf der Basis täglicher Messungen der Plasmaglucosekonzentration eingestellt wird.

5. Pharmazeutische Kombination zur Verwendung nach einem der vorhergehenden Ansprüche, wobei die in den Schritten (i) oder/und (ii) zu verabreichende Menge an Verbindung (b) so eingestellt wird, dass ein Nüchternplasmaglucosespiegel von etwa 4,4 mmol/l bis etwa 5,6 mmol/l oder/und ein selbstüberwachter Plasmaglucosespiegel von etwa 8 mmol/l erreicht oder zumindest nahezu erreicht wird.

6. Pharmazeutische Kombination zur Verwendung nach einem der vorhergehenden Ansprüche, wobei der zu behandelnde Patient übergewichtig ist und wobei der zu behandelnde Patient vorzugsweise einen Körpermasseindex von mindestens 30 kg/m² hat.

7. Pharmazeutische Kombination zur Verwendung nach einem der vorhergehenden Ansprüche, wobei es sich bei dem zu behandelnden Patienten um einen erwachsenen Patienten handelt.

8. Pharmazeutische Kombination zur Verwendung nach einem der vorhergehenden Ansprüche, wobei der zu behandelnde Patient zu Beginn von Schritt (i) nicht mit einem Insulin oder/und einem pharmazeutisch akzeptablen Salz davon behandelt wird.

9. Pharmazeutische Kombination zur Verwendung nach einem der vorhergehenden Ansprüche, wobei bei dem zu behandelnden Patienten Diabetes mellitus vom Typ 2 mindestens 1 Jahr oder mindestens 2 Jahre vor Beginn der Therapie diagnostiziert wurde.

10. Pharmazeutische Kombination zur Verwendung nach einem der vorhergehenden Ansprüche, wobei der zu behandelnde Patient einen HbA_{1c}-Wert von etwa 7 bis etwa 10% hat.

11. Pharmazeutische Kombination zur Verwendung nach einem der vorhergehenden Ansprüche, wobei der Patient zu Beginn von Schritt (i) eine Nüchternplasmaglucosekonzentration von mindestens 8 mmol/l hat.

12. Pharmazeutische Kombination zur Verwendung nach einem der vorhergehenden Ansprüche, wobei der Patient zu Beginn von Schritt (i) eine 2-Stunden-postprandiale Plasmaglucosekonzentration von mindestens 14 mmol/l hat.

13. Pharmazeutische Kombination zur Verwendung nach einem der vorhergehenden Ansprüche, wobei (a) das desPro³⁶Exendin-4(1-39)-Lys₆-NH₂ oder/und das pharmazeutisch akzeptable Salz davon für die parenterale Verabreichung zubereitet ist, (b) das Insulin glargin oder/und das pharmazeutisch akzeptable Salz davon für die parenterale Verabreichung zubereitet ist oder/und (c) das Metformin oder/und das pharmazeutisch akzeptable Salz davon für die orale Verabreichung zubereitet ist.

## Revendications

1. Combinaison pharmaceutique pour utilisation dans le traitement d'un patient atteint de diabète de type 2, où le diabète de type 2 est régulé de façon insuffisante par au moins un médicament antidiabétique oral, ladite combinaison comprenant :
(a) de la desPro³⁶Exendine-4(1-39)-Lys₆-NH₂ et/ou un sel pharmaceutiquement acceptable de celle-ci,
(b) de l'insuline glargine et/ou un sel pharmaceutiquement acceptable de celle-ci, et
(c) de la metformine et/ou un sel pharmaceutiquement acceptable de celle-ci, où le traitement du patient atteint de diabète de type 2 comprend les étapes consistant à :
(i) administrer les composés (b) et (c) pendant au moins 4 semaines, à la condition que le composé (a) ne soit pas administré, et
(ii) poursuivre le traitement par administration des composés (a), (b) et (c),
où au début de l'étape (i), le patient présente une glycémie plasmatique postprandiale à 2 heures d'au moins 12 mmol/L, et une excursion glycémique d'au moins 5 mmol/L, où l'excursion glycémique est la différence entre la glycémie plasmatique postprandiale à 2 heures et la glycémie plasmatique 30 minutes avant un test de repas, et où la quantité du composé (b) à administrer dans les étapes (i) et/ou (ii) est ajustée de sorte qu'une glycémie plasmatique à jeun prédéterminée et/ou une glycémie plasmatique auto-suivie prédéterminée soit atteinte au moins approximativement.

2. Combinaison pharmaceutique pour utilisation selon la revendication 1, où l'étape (i) comprend l'administration des composés (b) et (c) pendant au moins 4 semaines, au moins 8 semaines, au moins 12 semaines ou au moins 16 semaines.

3. Combinaison pharmaceutique pour utilisation selon l'une quelconque des revendications précédentes, où les étapes (i) et/ou (ii) comprennent en outre l'administration d'une thiazolidinedione.

4. Combinaison pharmaceutique pour utilisation selon l'une quelconque des revendications précédentes, où la quantité de composé (b) à administrer dans les étapes (i) et/ou (ii) est ajustée sur la base de mesures quotidiennes de glycémie plasmatique.

5. Combinaison pharmaceutique pour utilisation selon l'une quelconque des revendications précédentes, où la quantité de composé (b) à administrer dans les étapes (i) et/ou (ii) est ajustée de sorte qu'une glycémie plasmatique à jeun comprise entre environ 4,4 mmol/l et environ 5,6 mmol/l et/ou une glycémie plasmatique auto-suivie d'environ 8 mmol/l soit atteinte au moins approximativement.

6. Combinaison pharmaceutique pour utilisation selon l'une quelconque des revendications précédentes, où le patient à traiter est obèse, et où le patient à traiter présente préférentiellement un indice de masse corporelle d'au moins 30 kg/m².

7. Combinaison pharmaceutique pour utilisation selon l'une quelconque des revendications précédentes, où le patient à traiter est un patient adulte.

8. Combinaison pharmaceutique pour utilisation selon l'une quelconque des revendications précédentes, où le patient à traiter ne reçoit pas de traitement par une insuline et/ou un sel pharmaceutiquement acceptable de celle-ci au début de l'étape (i).

9. Combinaison pharmaceutique pour utilisation selon l'une quelconque des revendications précédentes, où chez le patient à traiter, un diabète sucré de type 2 a été diagnostiqué au moins 1 an ou au moins 2 ans avant le démarrage du traitement.

10. Combinaison pharmaceutique pour utilisation selon l'une quelconque des revendications précédentes, où le patient à traiter présente une valeur de HbA_{1c} comprise entre environ 7 et environ 10 %.

11. Combinaison pharmaceutique pour utilisation selon l'une quelconque des revendications précédentes, où au début de l'étape (i), le patient présente une glycémie plasmatique à jeun d'au moins 8 mmol/L.

12. Combinaison pharmaceutique pour utilisation selon l'une quelconque des revendications précédentes, où au début de l'étape (i), le patient présente une glycémie plasmatique postprandiale à 2 heures d'au moins 14 mmol/L.

13. Combinaison pharmaceutique pour utilisation selon l'une quelconque des revendications précédentes, où (a) la desPro³⁶Exendine-4(1-39)-Lys₆-NH₂ et/ou le sel pharmaceutiquement acceptable de celle-ci sont préparés pour administration parentérale, (b) l'insuline glargine et/ou le sel pharmaceutiquement acceptable de celle-ci sont préparés pour administration parentérale, et/ou (c) la metformine et/ou le sel pharmaceutiquement acceptable de celle-ci sont préparés pour administration orale.
